# EUROPEAN PATENT SPECIFICATION

(11) **EP 2 348 105 B1**
(45) Date of publication and mention of the grant of the patent: **24.10.2018**
(21) Application number: 10075534.7
(22) Date of filing: 26.09.2005
(51) Int. Cl.: C12N 5/0775

(54) **Multipotential expanded mesenchymal precursor cell progeny (MEMP) and uses thereof**
Multipotentiell verbesserte Mesenchymalvorläuferzellenvermehrung (MEMP) und Verwendungen dafür
Descendance cellulaire de précurseurs mesenchymateux développés multipotents (MEMP) et son utilisation

(30) Priority: 24.09.2004 AU 2004905528; 24.09.2004 AU 2004905525; 24.09.2004 AU 2004905526; 24.09.2004 AU 2004905527; 24.09.2004 US 613021 P; 19.10.2004 AU 2004906063; 19.10.2004 AU 2004906062; 19.10.2004 AU 2004906061; 19.10.2004 AU 2004906060
(43) Date of publication of application: 27.07.2011
(62) Divisional of application: 05787106.3
(73) Proprietor: Mesoblast, Inc., New York, NY 10017 (US)
(72) Inventor: Gronthos, Stan, Adelaide, South Australia 5041 (AU); Zannettino, Andrew Christopher William, Highbury, South Australia 5089 (AU)
(74) Representative: Harrison IP Limited

(56) References cited:
- WO-A2-2004/052177
- GRONTHOS STAN ET AL: "MOLECULAR AND CELLULAR CHARACTERISATION OF HIGHLY PURIFIED STROMAL STEM CELLS DERIVED FROM HUMAN BONE MARROW", JOURNAL OF CELL SCIENCE, CAMBRIDGE UNIVERSITY PRESS, LONDON, GB, vol. 116, no. 9, 1 May 2003 (2003-05-01), pages 1827-1835, XP009083626, ISSN: 0021-9533, DOI: DOI:10.1242/JCS.00369
- CASSIEDE P ET AL: "OSTEOCHONDROGENIC POTENTIAL OF MARROW MESENCHYMAL PROGENITOR CELLS EXPOSED TO TGF-BETA1 OR PDGF-BB AS ASSAYED IN VIVO AND IN VITRO", JOURNAL OF BONE AND MINERAL RESEARCH, AMERICAN SOCIETY FOR BONE AND MINERAL RESEARCH, NEW YORK, NY, US, vol. 11, no. 9, 1 January 1996 (1996-01-01), pages 1264-1273, XP000981538, ISSN: 0884-0431
- MOORE M A ET AL: "Mobilization of endothelial and hematopoietic stem and progenitor cells by adenovector-mediated elevation of serum levels of SDF-1, VEGF, and angiopoietin-1.", ANNALS OF THE NEW YORK ACADEMY OF SCIENCES JUN 2001 LNKD- PUBMED:11458524, vol. 938, June 2001 (2001-06), pages 36-45 ; DISC, XP002636294, ISSN: 0077-8923
- YU X ET AL: "STROMAL CELL-DERIVED FACTOR-1 (SDF-1) RECRUITS OSTEOCLAST PRECURSORS BY INDUCING CHEMOTAXIS, MATRIX METALLOPROTEINASE-9 (MMP-9) ACTIVITY, AND COLLAGEN TRANSMIGRATION", JOURNAL OF BONE AND MINERAL RESEARCH, AMERICAN SOCIETY FOR BONE AND MINERAL RESEARCH, NEW YORK, NY, US, vol. 18, no. 8, 1 August 2003 (2003-08-01) , pages 1404-1418, XP009034075, ISSN: 0884-0431, DOI: DOI:10.1359/JBMR.2003.18.8.1404
- WYNN R F ET AL: "A small proportion of mesenchymal stem cells strongly expresses functionally active CXCR4 receptor capable of promoting migration to bone marrow", BLOOD, AMERICAN SOCIETY OF HEMATOLOGY, US, vol. 104, no. 9, 13 July 2004 (2004-07-13) , pages 2643-2645, XP003019020, ISSN: 0006-4971, DOI: DOI:10.1182/BLOOD-2004-02-0526
- HODOHARA KEIKO ET AL: "Stromal cell-derived factor-1 (SDF-1) acts together with thrombopoietin to enhance the development of megakaryocytic progenitor cells (CFU-MK)", BLOOD, vol. 95, no. 3, 1 February 2000 (2000-02-01), pages 769-775, XP002635948, ISSN: 0006-4971
- PONOMARYOV TANYA ET AL: "Induction of the chemokine stromal-derived factor-1 following DNA damage improves human stem cell function", JOURNAL OF CLINICAL INVESTIGATION, vol. 106, no. 11, December 2000 (2000-12), pages 1331-1339, XP002635949, ISSN: 0021-9738
- MAGDA KUCIA ET AL: "CXCR4-SDF-1 Signalling, Locomotion, Chemotaxis and Adhesion", THE HISTOCHEMICAL JOURNAL, KLUWER ACADEMIC PUBLISHERS, DO, vol. 35, no. 3, 1 March 2004 (2004-03-01), pages 233-245, XP019243994, ISSN: 1573-6865
- KUCIA MAGDA ET AL: "Tissue-specific muscle, neural and liver stem/progenitor cells reside in the bone marrow, respond to an SDF-1 gradient and are mobilized into peripheral blood during stress and tissue injury.", BLOOD CELLS MOLECULES & DISEASES, vol. 32, no. 1, January 2004 (2004-01), pages 52-57, XP002636295, ISSN: 1079-9796
- GRONTHOS S ET AL: "The growth factor requirements of STRO-1-positive human bone marrow stromal precursors under serum-deprived conditions in vitro", BLOOD, AMERICAN SOCIETY OF HEMATOLOGY, US, vol. 85, no. 4, 15 February 1995 (1995-02-15), pages 929-940, XP002402196, ISSN: 0006-4971
- MAJUMDAR MANAS K ET AL: "Characterization and functionality of cell surface molecules on human mesenchymal stem cells.", JOURNAL OF BIOMEDICAL SCIENCE, vol. 10, no. 2, March 2003 (2003-03), pages 228-241, XP009164891, ISSN: 1021-7770
- MAJUMDAR MANAS KUMAR ET AL: "BMP-2 and BMP-9 promote chondrogenic differentiation of human multipotential mesenchymal cells and overcome the inhibitory effect of IL-1", JOURNAL OF CELLULAR PHYSIOLOGY, vol. 189, no. 3, December 2001 (2001-12), pages 275-284, XP009164892, ISSN: 0021-9541
- GRONTHOS S ET AL: "Differential cell surface expression of the STRO-1 and alkaline phosphatase antigens on discrete developmental stages in primary cultures of human bone cells", JOURNAL OF BONE AND MINERAL RESEARCH, AMERICAN SOCIETY FOR BONE AND MINERAL RESEARCH, NEW YORK, NY, US, vol. 14, no. 1, 1 January 1999 (1999-01-01), pages 47-56, XP008092055, ISSN: 0884-0431, DOI: 10.1359/JBMR.1999.14.1.47
- PAN BEIQING ET AL: "The nitrogen-containing bisphosphonate, zoledronic acid, increases mineralisation of human bone-derived cells in vitro", BONE, PERGAMON PRESS., OXFORD, GB, vol. 34, no. 1, 1 January 2004 (2004-01-01), pages 112-123, XP002458837, ISSN: 8756-3282, DOI: 10.1016/J.BONE.2003.08.013

## Description

### FIELD OF THE INVENTION

This invention relates to multipotential expanded mesenchymal precursor cell progeny (MEMPs). The present invention also relates to methods for producing MEMPs and to uses of MEMPs for therapeutic applications.

### BACKGROUND OF THE INVENTION

Non-hematopoietic progenitor cells that reside in the body and give rise to multipotential cells when isolated are referred to as Mesenchymal Precursor Cells (MPCs). More specifically, purified MPCs are capable of forming very large numbers of multipotential cell colonies.

Simmons et al. (Advances in Bone Marrow Purging and Processing: Fourth International Symposium, pages 271-280, 1994) describes enrichment of MPCs from freshly harvested bone marrow cells by selecting for cells that express the STRO-1 cell surface marker. As explained by the authors at pages 272-273, it is known that bone marrow cells contain a proportion of MPCs that are capable of giving rise to CFU-F. These CFU-F in turn are capable of giving rise under appropriate conditions to a broad spectrum of fully differentiated connective tissue, including cartilage, bone, adipose tissue, fibrous tissue and myelosupportive stroma.

MPCs and CFU-F are typically present at a very low incidence in bone marrow cells (typically between 0.05%-0.001%) and this rarity has been a major limitation to their study in the past. An important finding discussed in the Simmons *et al,* 1994 (supra) citation was the identification that these MPCs could be enriched from freshly isolated bone marrow cells to some extent by selecting for STRO-1 positive cells. In particular, the selection of STRO-1 positive cells enabled isolation of MPCs (and resultant CFU-F) free of contaminating hemopoietic progenitors.

WO 01/04268 (Simmons et al) provided a further important advance in the enrichment of MPCs by identifying a subpopulation within this fraction of STRO-1 positive cells that contains MPCs. In particular, WO 01/04268 describes the sorting of the STRO-1 positive cell population into three subsets: STRO-1^{dull}, STRO-1^{intermediate} and STRO-1^{bright}. Clonogenic assays for CFU-F in the different sorted subpopulations demonstrated that the vast majority of the MPCs are contained within the STRO-1^{bright} fraction.

WO 2004/085630 (Gronthos et al) discloses for the first time that MPCs are present in perivascular tissue. One of the benefits of this finding is that it greatly expands the range of source tissues from which MPCs can be isolated or enriched and there is no longer an effective restriction on the source of MPCs to bone marrow. The tissues from which MPCs can isolated according to the methods described in WO 2004/085630 include human bone marrow, dental pulp, adipose tissue, skin, spleen, pancreas, brain, kidney, liver and heart. The MPCs isolated from perivascular tissue are positive for the cell surface marker 3G5. They can therefore be isolated by enriching for cells carrying the 3G5 marker, or by enriching for an early developmental surface marker present on perivascular cells such as CD146 (MUC18), VCAM-1, or by enriching for high level expression of the cell surface marker STRO-1.

Methods for propagating isolated MPCs *in vitro* have been described (Gronthos et al. Journal of Cell Science 116: 1827-1835, 2003). The generally accepted view, however, is that expansion of MPCs *in vitro* results in the loss of their progenitor nature through differentiation.

Gronthos et al. (Blood, American Society of Hematology, US, vol.85, no.4, 929-940, Feb 1995) discloses a population of adult bone marrow stromal stem cells (BMSSCs) or mesenchymal stem cells. The cells express the Stro-1 marker. The cells are stimulated by the addition of a combination of EGF abd PDGF-BB.

### SUMMARY OF THE INVENTION

The present inventors have now made the surprising finding that *ex vivo* expanded MPCs give rise a sub population of progeny that retain multipotentiality. This subpopulation of MPC progeny are Stro-1^{bri} cells and are referred to herein as Multipotential Expanded MPC Progeny (MEMPs).

The present inventors have also made the surprising finding that MEMPs are capable of stimulating proliferation of tissue specific committed cells (TSCCs) both *in vitro* and *in vivo.* Thus, MEMPs have potential use in a wide range of therapeutic applications where generation or repair of tissue is required.

Accordingly, the present invention provides an *in vitro* method of increasing the generation of multipotential expanded mesenchymal precursor cell progeny (MEMPs) that have the phenotype Stro-1^{bri}, ALP⁻, the method comprising culturing STRO-1^{bright} ALP⁺ mesenchymal progenitor cells (MPC) in the presence of one or more stimulatory factors selected from the group consisting of 1α,25-dihydroxyvitamin D₃ (1,25D), tumor necrosis factor α (TNF-α), and interleukin-1β (IL-1β).

### BRIEF DESCRIPTION OF THE FIGURES

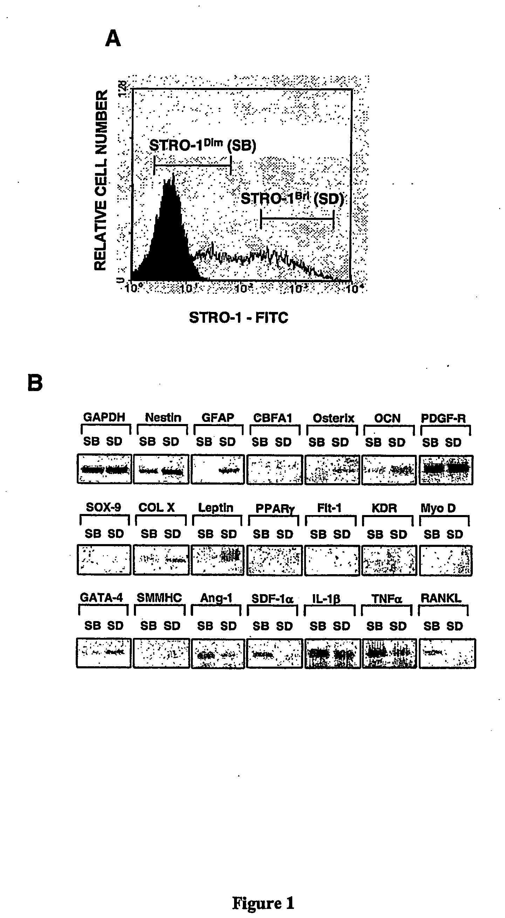

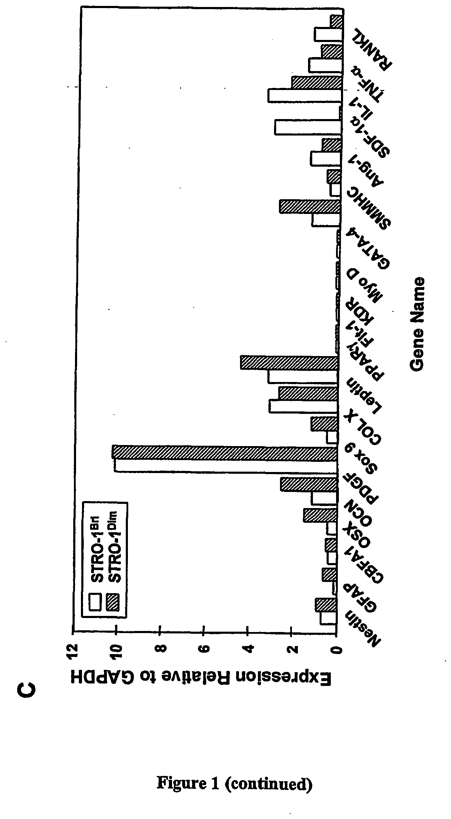

**Figure 1****. Gene expression profile of STRO-1^{bri} or STRO-1^{dim} expressing cells derived from cultured MPC.** Single cell suspensions of *ex vivo* expanded bone marrow MPC were prepared by trypsin/EDTA treatment. Cells were stained with the STRO-1 antibody which was subsequently revealed by incubation with goat-anti murine IgM-fluorescein isothiocyanate. Total cellular RNA was prepared from purified populations of STRO-1^{dim} or STRO-1^{bri} expressing cells, following fluorescence activated cell sorting (A). Using RNAzolB extraction method, and standard procedures, total RNA was isolated from each subpopulation and used as a template for cDNA synthesis. The expression of various transcripts was assessed by PCR amplification, using a standard protocol as described previously (Gronthos et al. Journal of Cell Science 116: 1827-1835, 2003). Primers sets used in this study are shown in Table 2. Following amplification, each reaction mixture was analysed by 1.5% agarose gel electrophoresis, and visualised by ethidium bromide staining (B). Relative gene expression for each cell marker was assessed with reference to the expression of the house-keeping gene, GAPDH, using ImageQant software (C).

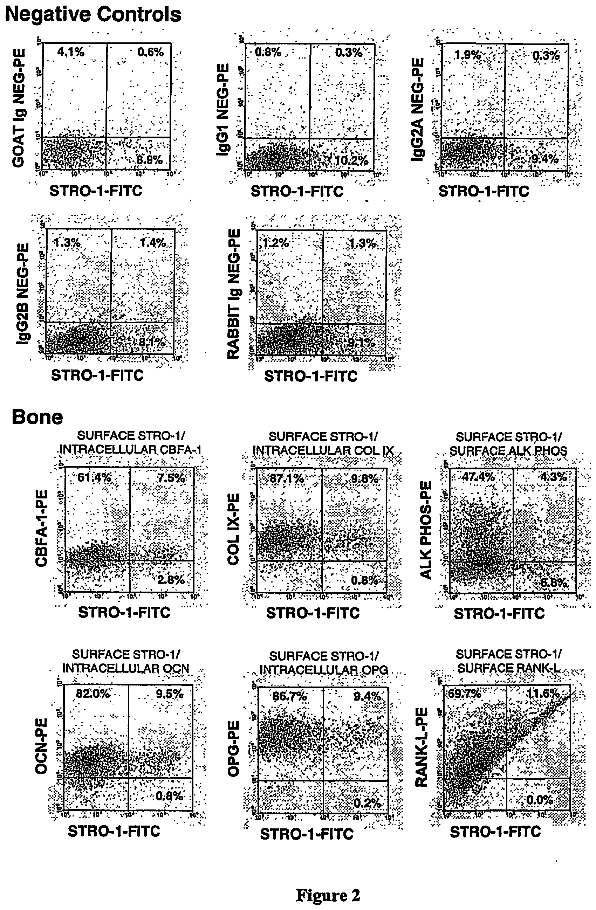

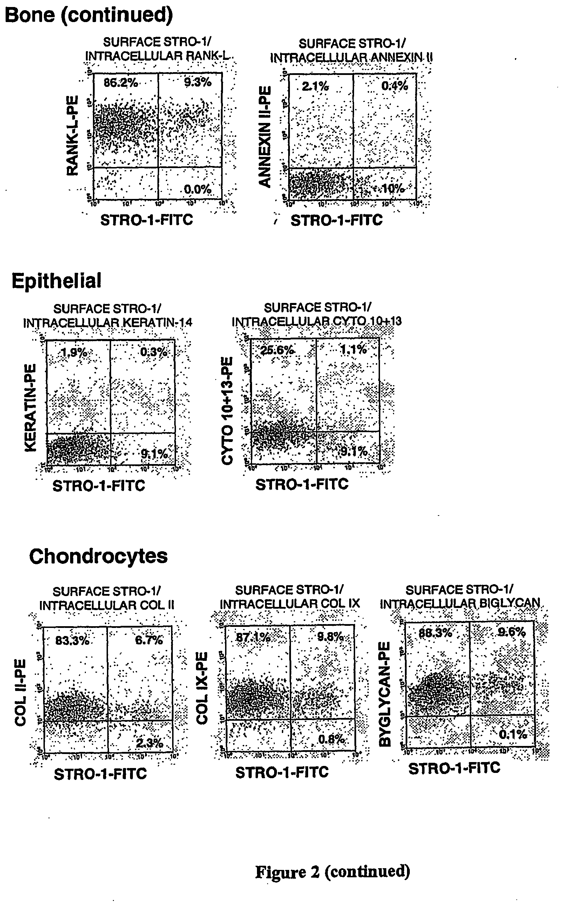

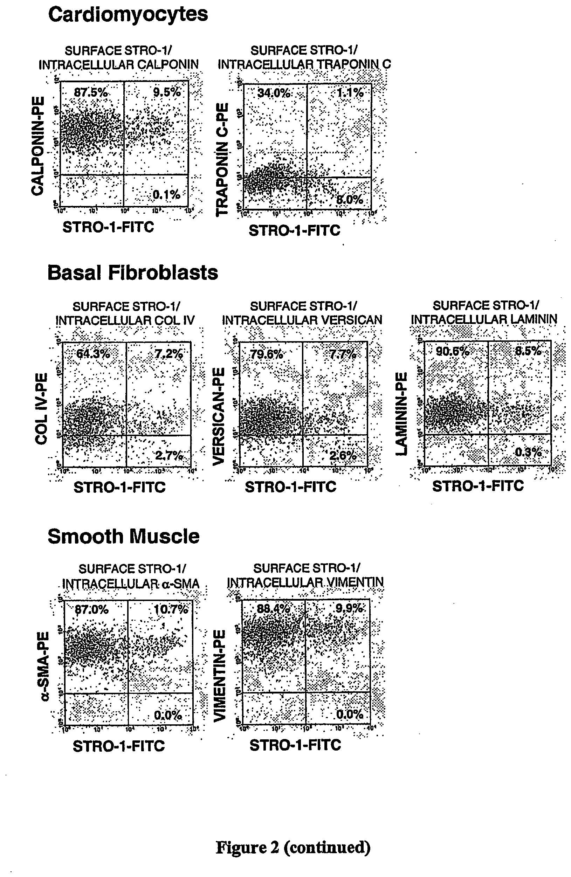

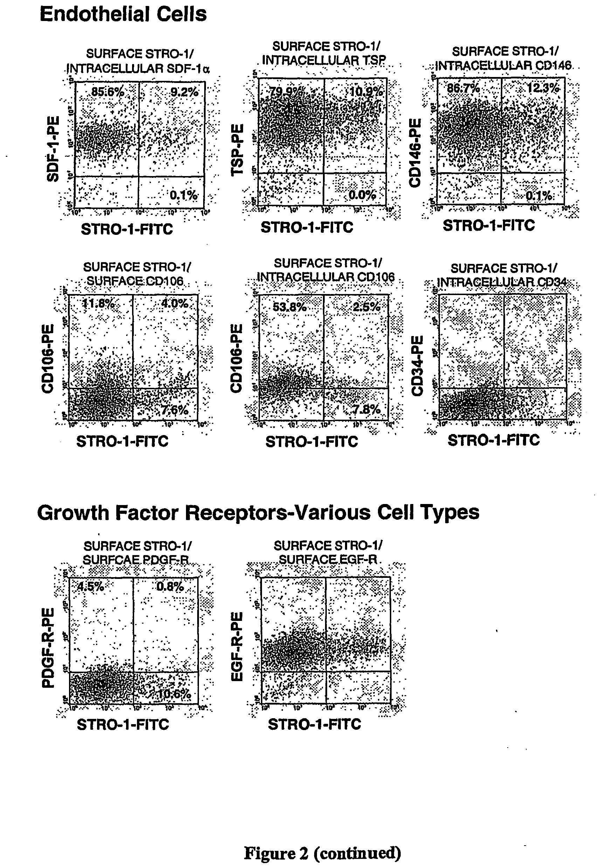

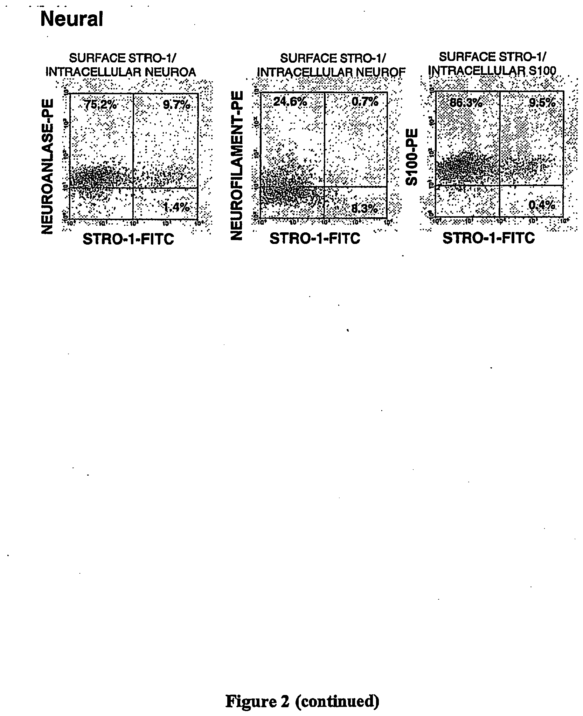

**Figure 2****. Immunophenotypic expression pattern of *ex vivo* expanded cells derived from bone marrow MPCs.** Single cell suspensions of *ex vivo* expanded cells derived bone marrow MPC were prepared by trypsin/EDTA detachment and subsequently incubated with the STRO-1 antibody in combination with antibodies identifying a wide range of cell lineage-associated markers. STRO-1 was identified using a goat anti-murine IgM-fluorescein isothiocyanate while all other markers were identified using either a goat anti-mouse or anti-rabbit IgG- phycoerythrin. For those antibodies identifying intracellular antigens, cell preparations were first labelled with the STRO-1 antibody, fixed with cold 70% ethanol to permeabilize the cellular membrane and then incubated with intracellular antigen-specific antibodies. Isotype matched control antibodies were used under identical conditions. Dual-colour flow cytometric analysis was performed using a COULTER EPICS flow cytometer and list mode data collected. The dot plots represent 5,000 listmode events indicating the level of fluorescence intensity for each lineage cell marker (y-axis) and STRO-1 (x-axis). The vertical and horizontal quadrants were established with reference to the isotype matched negative control antibodies.

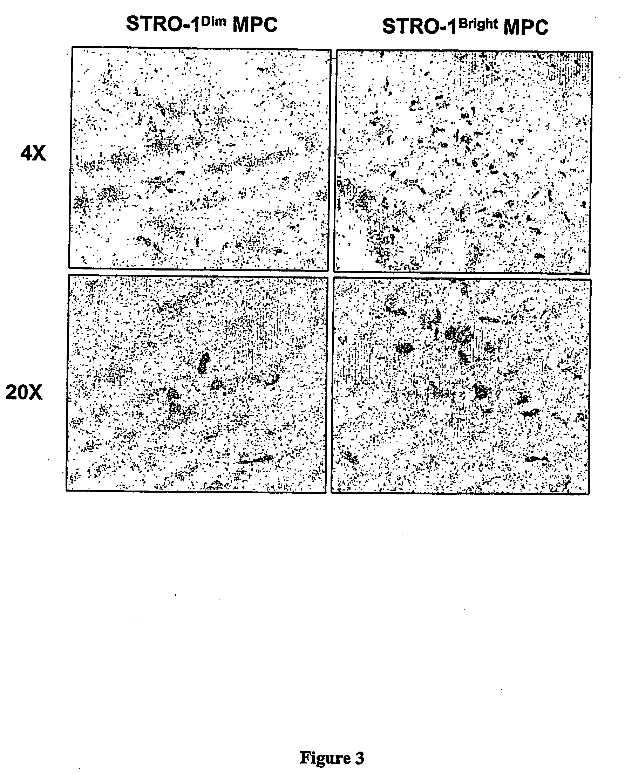

**Figure 3****. Adipogenic Development *In Vitro.*** Single cell suspensions were generated by trypsin/EDTA digest from secondary cultures of *ex vivo* expanded cells, derived from STRO-1^{bri}/VCAM-1⁺ sorted bone marrow cells. The expanded cells were then isolated according to their expression of STRO-1 using single colour fluorescence activated cell sorting as shown in Figure 1A. STRO-1^{bri} and STRO-1^{dim} sorted MPC derived cells were then plated overnight, into 6-well plates, at a density of 1 x 10⁵ cells per well under regular growth medium. On the following day the culture medium was replaced with adipogenic inductive medium as described in the methods. The cultures were fed twice a week thereafter for a total period of three weeks at which time the cells were fixed and stained with Oil red O. Low (4x) and high (20x) power magnifications are shown depicting Oil red O staining of lipid containing adipocytes scattered throughout the adherent stromal layers. On average 22±5 Oil red O positive adipocytes were identified in the STRO-1^{bri} cultures (per unit area at 20x, n=9 fields) when compared to 7±2 adipocytes (per unit area at 20x, n=9 fields) in the STRO-1^{dim} cultures.

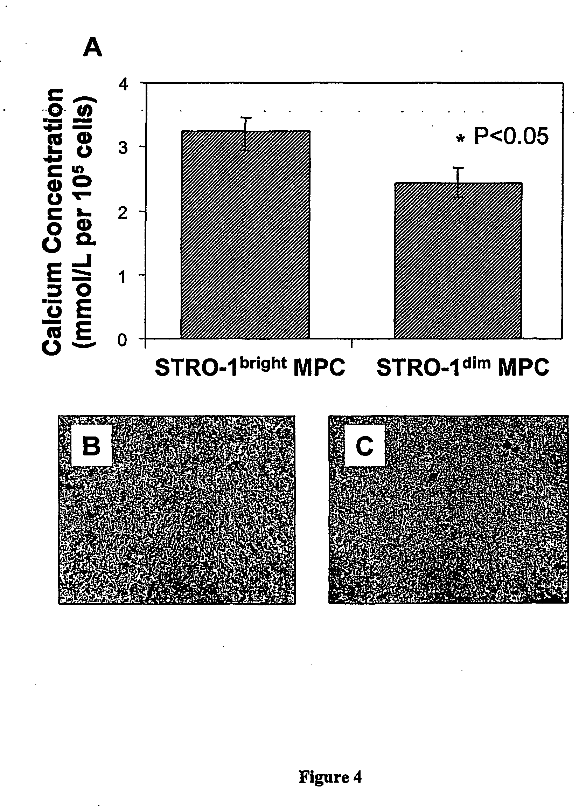

**Figure 4****. Osteogenic Development *In Vitro.*** Single cell suspensions were generated by trypsin/EDTA digest from secondary cultures of *ex vivo* expanded cells, derived from STRO-1^{bri}/VCAM-1⁺ sorted bone marrow cells. The expanded cells were then isolated according to their expression of STRO-1 using single colour fluorescence activated cell sorting (FACS) as shown in Figure 1A. STRO-1^{bri} and STRO-1^{dim} FACS isolated cells were then plated overnight, into 48-well plates, at a density of 0.3 X 10⁵ cells per well under regular growth medium (four replicates per condition). On the following day the culture medium was replaced with osteogenic inductive medium as described in the methods. The cultures were fed twice a week thereafter for a total period of three weeks at which time the cells were washed then treated with 0.6N HCl to extract the calcium within the mineralized deposits. Samples were reacted with o-cresol-phthalein-complexon and the colorimetric reaction was read at 570 nm. The absolute calcium concentration was determined according to a standard curve for calcium. (A) Calcium measurements showed that the STRO-1^{bri} cultures synthesised significantly (*; p<0.05; t-test) more mineral when compared to the STRO-1^{dim} cultures. Replicate cultures were fixed and stained for Alizarin red staining depicting typical levels of mineralised deposits in the adherent layers of STRO-1^{bri} (B) and STRO-1^{dim} (C) cultures.

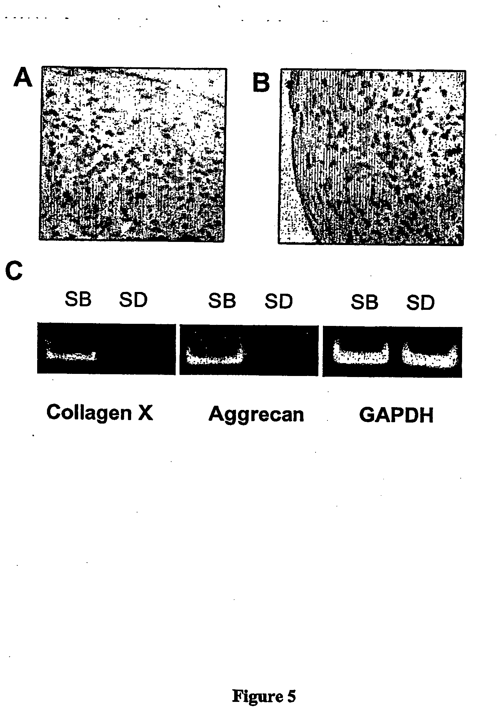

**Figure 5****. Chondrogenic Development *In Vitro.*** Single cell suspensions were generated by trypsin/EDTA digest from secondary cultures of *ex vivo* expanded cells, derived from STRO-1^{bri}NCAM-1⁺ sorted bone marrow cells. The expanded cells were then isolated according to their expression of STRO-1 using single colour fluorescence activated cell sorting (FACS) as shown in Figure 1A. STRO-1^{bri} and STRO-1^{dim} FACS isolated cells were then pelleted into polypropylene tubes at a density of 2.5 x 10⁵ cells per tube and cultured in chondrogenic inductive media. The cultures were fed twice a week thereafter for a total period of three weeks. Cell pellets were retrieved and used for histological examination or preparation of total RNA as described in the methods. Both STRO-1^{bri} (A) and STRO-1^{dim} (B) cell populations were capable of forming cell pellets containing chondrocyte-like cells. RT-PCR analysis indicated that the STRO-1^{bri} (SB) population demonstrated higher levels of the cartilage associated genes collagen type X and aggrecan when compared to the STRO-1^{dim} (SD) cell population (C).

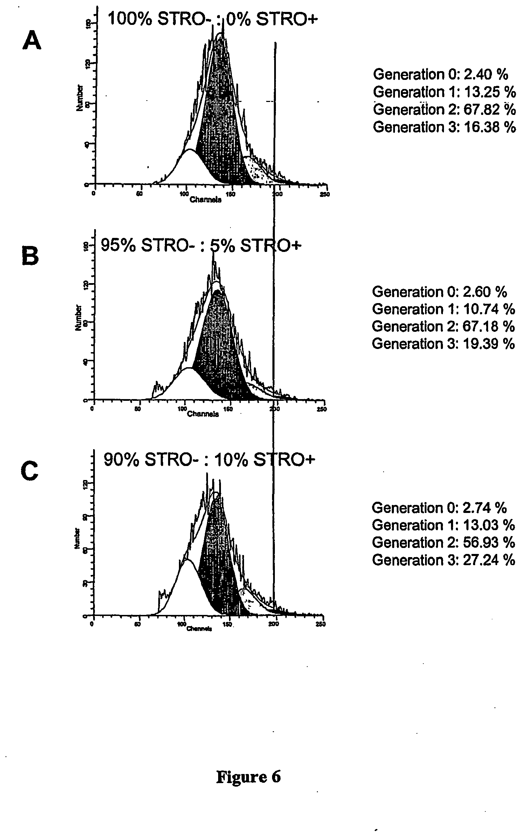

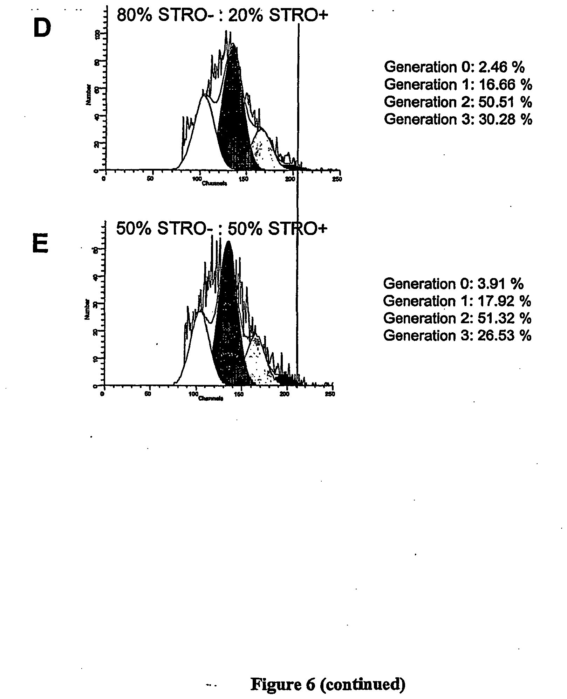

**Figure 6****. STRO-1^{bri} cells induce the proliferation of STRO-1^{dim} cells.** Single cell suspensions of *ex vivo* expanded bone marrow MPC were prepared by trypsin/EDTA treatment. Cells were stained with the STRO-1 antibody and sorted into populations of STRO-1^{dim} or STRO-1^{bri} expressing cultured cell populations as described in Figure 1. Cells were labelled with CFSE as described in the methods. Unlabelled cells were used to establish a negative control (auto-fluorescence). Colcemid® (100 ng/ml) was used to inhibit cell division and provided an input labelling index (Generation 0). Unlabelled STRO-1^{bri} were subsequently added back to the CFSE-labelled STRO-1^{dim} cells at a ratio of (A) 0 STRO-1^{bri} cells : 1x10⁵ STRO-1^{dim} cells (0%); (B) 0.05 x 10⁵ STRO-1^{bri} cells : 0.95x10⁵ STRO-1^{dim} cells (5%); (C) 0.1x10⁵ STRO-1^{bri} cells : 0.9x10⁵ STRO-1^{dim} cells (10%); (D) 0.2x10⁵ STRO-1^{bri} cells : 0.8x10⁵ STRO-1^{dim} cells (20%); (E) 0.5x10⁵ STRO-1^{bri} cells : 0.5x10⁵ STRO-1^{dim} cells (50%). The add-mixtures were cultured for a period of 7 days, harvested, and analysed by flow cytometry as described in the methods. Cell proliferation was analysed using the ModFit LT for win 32 (Version 2.0). The STRO-1^{bri} cells (R1) were found to stimulate the proliferation of STRO-1^{dim} cells in a dose-dependent manner.

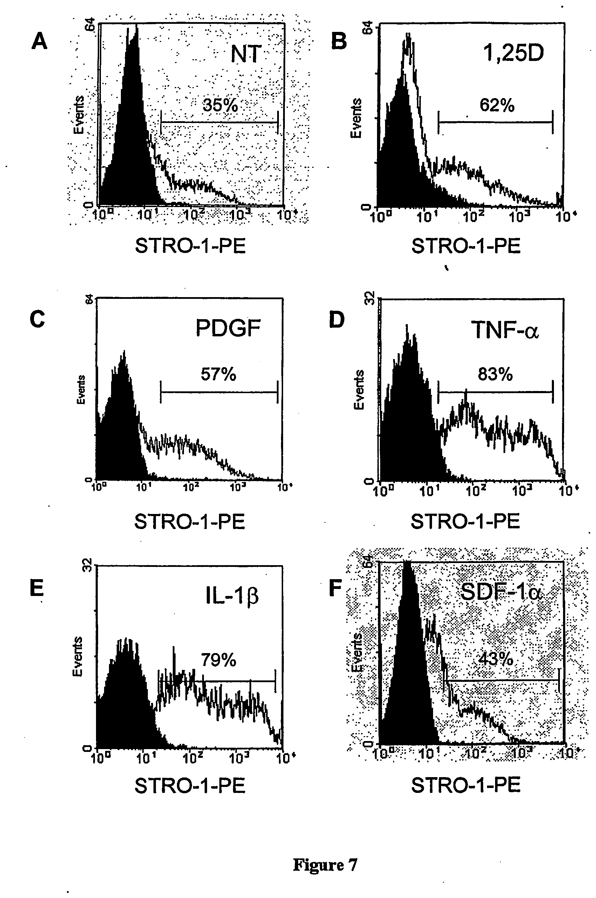

**Figure 7****. Cytokines and osteotropic agents increase the number of STRO-1^{bri} cells in culture.** Established cultures of MPC were cultured in basal medium supplemented with 10% FCS (A), or a range of factors, including 1x10⁻⁸M 1α,25-dihydroxyvitamin D3 (1,25D) (B), 10ng/ml Platelet derived growth factor (PDGF) (C), 10 ng/ml Tumor necrosis factor-alpha (TNF-α) (D); 10 ng/ml interleukin-1β (IL-1β) (E) and 30 ng/ml stromal derived factor 1-alpha (SDF-1α) (F), for 5 days, stained with STRO-1 mAb and analysed as described above. These factors were found to increase the number of STRO-1^{bri} MPC. The results displayed are a representative example of 3 independent experiments.

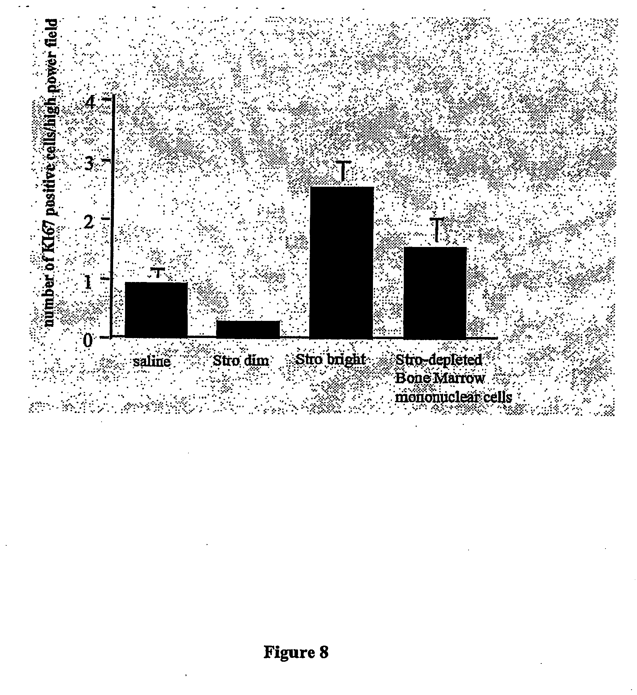

**Figure 8****.** Athymic nude rats underwent ligation of the left anterior descending (LAD) coronary artery and injected 48 hours later with saline, 1x10⁶ human Stro-1^{dim} cells, 1x10⁶ human Stro-1^{bri} cells or 1x10⁶ human Stro-1-depleted bone marrow mononuclear cells. Two weeks later, animals were sacrificed, and cardiac tissues were fixed and concomitantly stained with two monoclonal antibodies: the first being selectively reactive with the rat, but not the human, Ki67 antigen, and the second being reactive with the cardiomyocyte marker troponin I. Dually stained cells, indicative of proliferating rat cardiomyocytes, were detected by immunoperoxidase technique. Animals receiving 1x10⁶ Stro-1^{bri} human cells demonstrated 2.5-5 fold higher numbers of proliferating rat cardiomyocytes compared with control animals receiving saline or 1x10⁶ Stro-1^{dim} human cells (p<0.05).

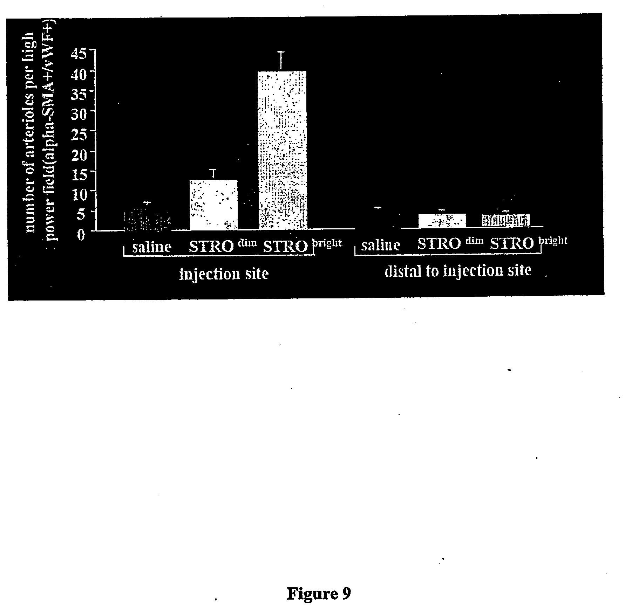

**Figure 9****.** Athymic nude rats were injected subcutaneously with rat glioblastoma tumor cells, which constitutively secrete VEGF. Two weeks later, the rats received intra-tumor injections with saline, 0.5x10⁶ human Stro-1^{dim} cells or 0.5x10⁶ human Stro-1^{bri} cells. One week later, animals were sacrificed, and tumor tissues were fixed and concomitantly stained with two monoclonal antibodies: the first being reactive with the alpha-smooth muscle actin antigen expressed by smooth muscle cells, and the second being reactive with the vWF antigen expressed by vascular endothelial cells. Dually stained structures, indicative of arterioles and arteries containing both endothelium and smooth muscle, were detected by immunoperoxidase technique. Animals receiving 0.5x10⁶ Stro-1^{bri} human cells demonstrated 3.5-8 fold higher numbers of arterioles and arteries at the site of cellular injection in the tumors compared with control animals receiving saline or 1x10⁶ Stro-1^{dim} human cells (p<0.05). No differences were seen at sites distal to where the human cells had been injected.

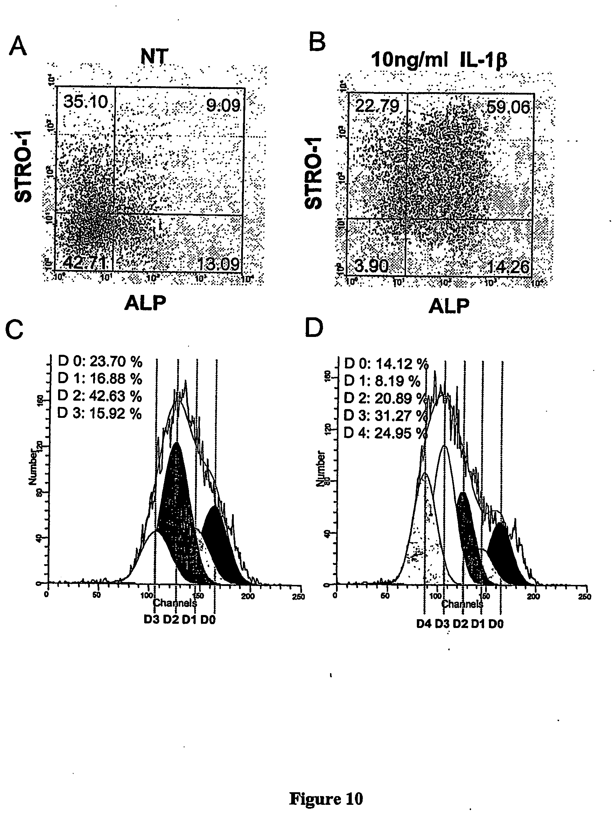

**Figure 10****. IL-1β increases the proliferative potential of cells expanded from MPC. Cells were labelled with CFSE as described in the methods.** Cells were subsequently cultured in the presence of 10 ng/ml IL-1β for 5 days, stained with STRO-1 and ALK PHOS mAb and analysed as described above. (A) non-treated (NT) and (B) IL-1β-treated cultures display an increase in the number of STRO-1^{bri} /ALP positive cells. This increase in STRO-1 expression is accompanied by an increase in cell proliferation as shown in (C) where untreated cultures have undergone four cell divisions, whilst (D) IL-1β treated cultures exhibit an increase in the number of cell divisions by increasing the number of STRO-1^{bri} osteoprogenitor cells. The results displayed are a representative example of 3 independent experiments. Similar results were also obtained 1,25D, PDGF-BB, TNF-α, IL-1β, and SDF-1α were used to stimulate MPCs.

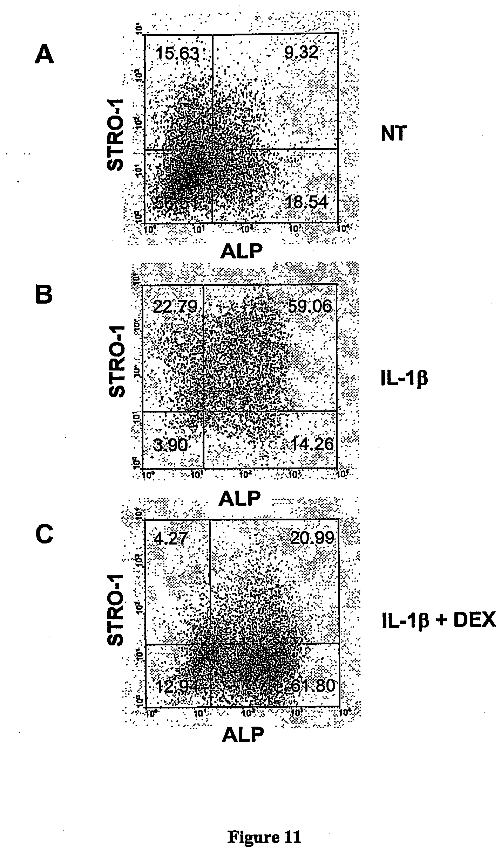

**Figure 11****. IL-1β stimulates MPC proliferation and enhances their bone forming potential in the presence of the osteoinductive agent, dexamethasone.** Human (A) *ex vivo* expanded progeny of MPC were seeded in 96-well plates at a cell density of 2,000 cells/ well and cultured in α-MEM-10. Cultures were supplemented with IL-1β at the indicated concentrations and the cell number and viability quantitated at d7 using WST-1, as described in the methods. IL-1β at concentration 0.01ng/ml significantly increased cell number to 136.6±1.2% of untreated control cultures (D, P=0.000003, Student t-test). A plateau effect was achieved at concentrations greater than 0.1 ng/ml. Values represent means ± SEM of triplicate cultures of each concentration. (B & C) *Ex vivo* expanded progeny of MPC were seeded into 24-well plates at a cell density of 5×10⁴/well in triplicate, and cultured in osteoinductive conditions, as described in the methods. The cells were treated with IL-1β at a concentration 10 ng/ml and cultures were "fed" weekly with fresh medium containing IL-1β. The release of free calcium from the matrix was achieved by treating the adherent cell layers under acidic condition as described in the methods. Samples were reacted with o-cresol-phthalein-complexon and the colorimetric reaction was read at 570 nm. The absolute calcium concentration was determined according to a standard curve for calcium. The results showed that mineral deposition was increased in cells treated with IL-1β (C) compared to untreated cells (B). The calcium level in IL-1β treated cells was significantly higher than that in untreated cells at both week 4 (***P*=0.00009, Student t-test) and week 6 (***P*=0.00004, Student t-test) (D). The results displayed are a representative example of 3 independent experiments, using stromal cells derived from three different donors.

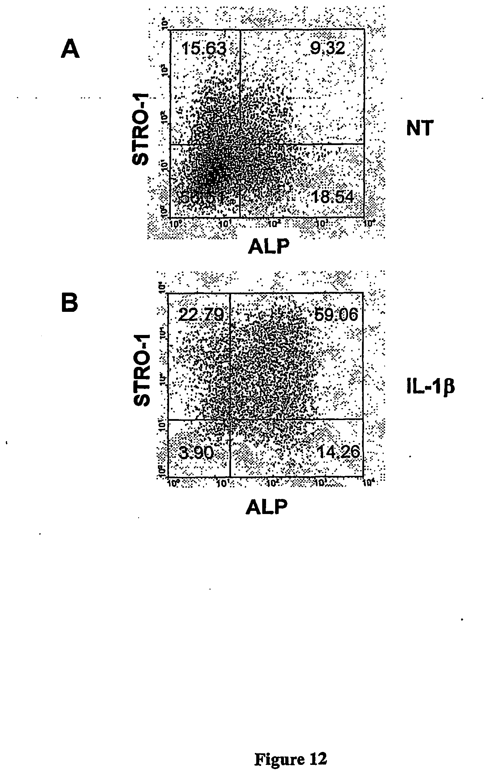

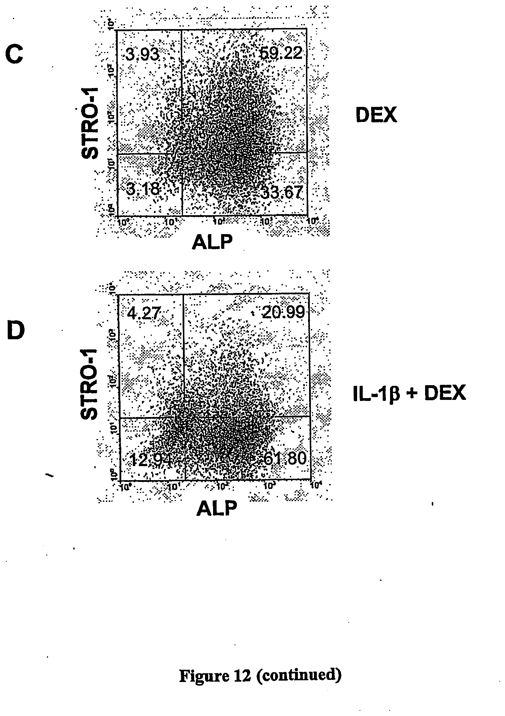

**Figure 12****. IL-1β stimulates the proliferation and STRO-1^{bri} MPC, whilst dexamethasone induces alkaline phosphatase (ALP) expression.** Established cultures of human MPC were seeded in a 24-well plate at a cell density of 3 x 10₄/well in complete medium supplemented with (A) nothing (NT), (B) 10ng/ml IL-1β or (C) 1x10⁻⁸M Dexamethasone and (D) 10ng/ml IL-1β and 1x10⁻⁸M Dexamethasone. Cells were cultured for 21 days as described in the methods. The results suggest that the mitogenic action of IL-1β serves to increase the number of STRO-1^{bri} MPC (B), which in turn stimulates the proliferation of the STRO-1^{dim} cells (see Figure 6). In addition, MPC acquire the expression of ALP in response to the FCS and ascorbate-2-phosphate present in the growth medium which is enhanced in response to the glucocorticosteroid, dexamethasone (dex) (D). The combined action of IL-1β and dex serve to enhance bone formation as seen in Figure 11. The experiments were performed three times and a similar trend was observed in MPC derived from three different donors.

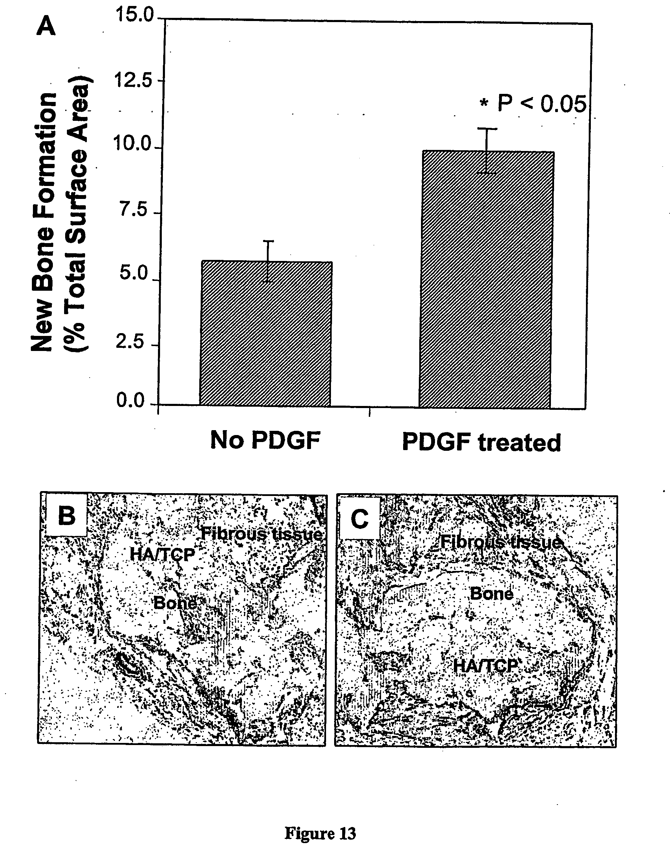

**Figure 13****. Effect of PDGF on Bone Formation *In Vivo.*** Semi-confluent secondary cultures of *ex vivo* expanded cells, derived from STRO-1^{bri}/VCAM-1⁺ sorted bone marrow cells, were cultured in the presence or absence of PDGF-BB (10ng/ml) for five days. Single cell suspensions were generated by trypsin/EDTA digest then incubated with 40mg of hydroxyapetite/tricalcium phosphate particles (HA/TCP) for implantation into immunocompromised mice as described in the methods. After eight weeks, the harvested transplants were fixed and processed for histological examination. Analysis of new bone formation was determined using Scion Imaging software per surface area (20x) from three replicate transplants (A). Cultures pre-treated with PDGF-BB demonstrated significantly (*; p<0.05; t-test) more ecotpic bone formation when compared to control untreated cultures. Typical images are shown depicting haematoxylin/eosin stained ectopic bone in cross-sections representative of untreated (B) and PDGF treated (C) transplants.

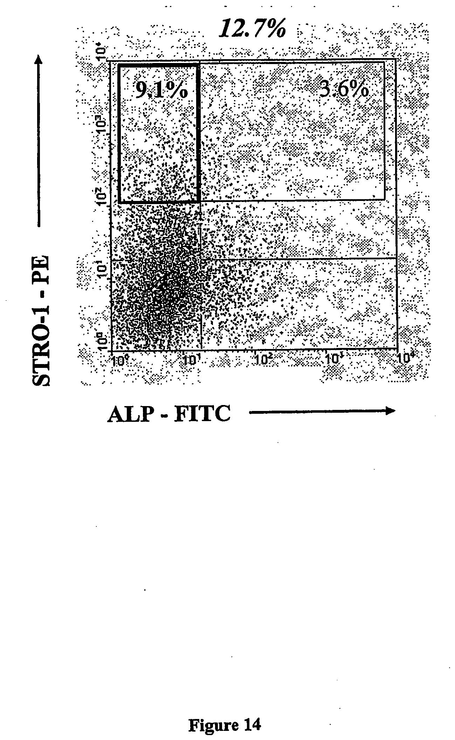

**Figure 14****: Multipotential expanded mesenchymal precursor cell progeny (MEMPs) or STRO-1^{bri} / ALP- MPC persist in *ex vivo* cultures of STRO-1 selected BM MPC.** Dual-colour immunofluorescence and flow cytometry examining STRO-1 and ALP expression was performed on STRO-1 selected BM MPC following 4 passages of *ex vivo* culture. The dot plot histogram represents 5 x 10⁴ events collected as listmode data. The vertical and horizontal lines were set to the reactivity levels of <1.0% mean fluorescence obtained with the isotype-matched control antibodies, 1B5 (IgG) and 1A6.12 (IgM) treated under the same conditions.

### DETAILED DESCRIPTION OF PREFERRED EMBODIMENTS OF THE INVENTION

The present inventors have now made the surprising finding that *ex vivo* expanded MPCs contain a sub population of cells that retain multipotentiality. More specifically, the inventors have found that expanded populations derived from harvested MPC cells can be separated into at least two populations on the basis of level of expression of the antigen recognised by the STRO-1 antibody into STRO-1^{bri} and STRO-1^{dim}. Functional data presented herein show that the expanded STRO-1^{bri} cells are less committed and more able to respond to inductive factors which support fat development, cartilage development and bone development. In contrast, the STRO-1^{dim} cells represent a more differentiated population and include Tissue Specific Committed Cell (TSCC) types. The Stro-1^{bri} cells within the expanded progeny are referred to herein as Multipotential Expanded MPC Progeny (MEMPs).

The present inventors have also made the surprising finding that MEMPs are capable of stimulating proliferation of tissue specific committed cells (TSCCs) both *in vitro* and *in vivo.* Thus, MEMPs have potential use in a wide range of therapeutic applications where generation or repair of tissue is required.

As used herein, "MPC" are non-hematopoietic progenitor cells that are capable of forming large numbers of multipotential cell colonies.

By "MPC progeny" we mean cells derived from MPC. Preferably the MPC progeny are progeny of colony forming units-fibroblast (CFU-F), which in turn are derived from MPC. More preferably, the cells are derived from MPC or CFU-F by expansion or culturing *ex vivo.* Preferably, the culturing involves more than two, preferably more than three and more preferably more than four passages. Following culturing or expansion it is preferred that the enriched population comprises at least 5 x 10⁶ cells, more preferably at least 10⁷ cells, and more preferably at least 10⁹ cells.

Methods for preparing enriched populations of MPC from which progeny may be derived are described in WO01/04268 and WO2004/085630. In an *in vitro* context MPCs will rarely be present as an absolutely pure preparation and will generally be present with other cells that are tissue specific committed cells (TSCCs). WO01/04268 refers to harvesting such cells from bone marrow at purity levels of about 0.1% to 90%.

The population comprising MPC from which progeny are derived may be directly harvested from a tissue source, or alternatively it may be a population that has already been expanded *ex vivo.*

For example, the progeny may be obtained from a harvested, unexpanded, population of substantially purified MPC, comprising at least about 0.1, 1, 5, 10, 20, 30, 40, 50, 60, 70, 80 or 95% of total cells of the population in which they are present. This level may be achieved, for example, by selecting for cells that are positive for at least one marker selected from the group consisting of STRO-1^{bri}, VCAM-1^{bri}, THY-1^{bri}, CD146^{bri} and STRO-2^{bri}.

The MPC starting population may be derived from any one or more tissue types set out in WO01/04268 or WO2004/085630, namely bone marrow, dental pulp cells, adipose tissue and skin, or perhaps more broadly from adipose tissue, teeth, dental pulp, skin, liver, kidney, heart, retina, brain, hair follicles, intestine, lung, spleen, lymph node, thymus, pancreas, bone, ligament, bone marrow, tendon and skeletal muscle.

The preferred source of such cells is human, however, it is expected that the invention is also applicable to animals, including agricultural animals such as cows, sheep, pigs and the like, domestic animals such as dogs and cats, laboratory animals such as mice, rats, hamsters and rabbits or animals that are be used for sport such as horses.

It will be understood that in performing the present invention, separation of cells carrying any given cell surface marker can be effected by a number of different methods, however, preferred methods rely upon binding a binding agent to the marker concerned followed by a separation of those that exhibit binding, being either high level binding, or low level binding or no binding. The most convenient binding agents are antibodies or antibody based molecules, preferably being monoclonal antibodies or based on monoclonal antibodies because of the specificity of these latter agents. Antibodies can be used for both steps, however other agents might also be used, thus ligands for these markers may also be employed to enrich for cells carrying them, or lacking them.

The antibodies or ligands may be attached to a solid support to allow for a crude separation. The separation techniques preferably maximise the retention of viability of the fraction to be collected. Various techniques of different efficacy may be employed to obtain relatively crude separations. The particular technique employed will depend upon efficiency of separation, associated cytotoxicity, ease and speed of performance, and necessity for sophisticated equipment and/or technical skill. Procedures for separation may include, but are not limited to, magnetic separation, using antibody-coated magnetic beads, affinity chromatography and "panning" with antibody attached to a solid matrix. Techniques providing accurate separation include but are not limited to FACS.

It is preferred that the method for isolating MPCs, for example, comprises a first step being a solid phase sorting step utilising for example MACS recognising high level expression of STRO-1. A second sorting step can then follow, should that be desired, to result in a higher level of precursor cell expression as described in patent specification WO 01/14268. This second sorting step might involve the use of two or more markers.

The method obtaining MPCs might also include the harvesting of a source of the cells before the first enrichment step using known techniques. Thus the tissue will be surgically removed. Cells comprising the source tissue will then be separated into a so called single cells suspension. This separation may be achieved by physical and or enzymic means.

Once a suitable MPC population has been obtained, it may be cultured or expanded by any suitable means to obtain MEMPs.

MEMPS can be distinguished from freshly harvested MPCs and that they are positive for the marker STRO-1^{bri} and negative for the marker Alkaline phosphatase (ALP). In contrast, freshly isolated MPCs are positive for both STRO-1^{bri} and ALP.

When we refer to a cell as being "positive" for a given marker it may be either a low (lo or dim) or a high (bright, bri) expresser of that marker depending on the degree to which the marker is present on the cell surface, where the terms relate to intensity of fluorescence or other colour used in the colour sorting process of the cells. The distinction of lo and bri will be understood in the context of the marker used on a particular cell population being sorted. When we refer herein to a cell as being "negative" for a given marker, it does not mean that the marker is not expressed at all by that cell. It means that the marker is expressed at a relatively very low level by that cell, and that it generates a very low signal when detectably labelled.

The term "bright", when used herein, refers to a marker on a cell surface that generates a relatively high signal when detectably labelled. Whilst not wishing to be limited by theory, it is proposed that "bright" cells express more of the target marker protein (for example the antigen recognised by STRO-1) than other cells in the sample. For instance, STRO-1^{bri} cells produce a greater fluorescent signal, when labelled with a FITC-conjugated STRO-1 antibody as determined by FACS analysis, than non-bright cells (STRO-1^{dull/dim}). Preferably, "bright" cells constitute at least about 0.1% of the most brightly labelled bone marrow mononuclear cells contained in the starting sample. In other embodiments, "bright" cells constitute at least about 0.1%, at least about 0.5%, at least about 1%, at least about 1.5%, or at least about 2%, of the most brightly labelled bone marrow mononuclear cells contained in the starting sample.

Accordingly, the present invention may provide an enriched cell population wherein at least 10% of the total cell population are Multipotential Expanded Mesenchymal Precursor Cell Progeny (MEMPs) that have the phenotype STRO-1^{bri}, ALP⁻.

In a preferred embodiment, at least 15%, 20%, 30%, 40%, 50%, 60%, 70%, 80%, 90% or 95% of the total enriched cell population are MEMPs that have the phenotype STRO-1^{bri}, ALP⁻.

In another preferred embodiment, the enriched cell population is homogenous for MEMPs that have the phenotype STRO-1^{bri}, ALP⁻.

In a further preferred embodiment the MEMPS are positive for one or more of the markers Ki67, CD44 and/or CD49c/CD29, VLA-3, α3β1.

In yet a further preferred embodiment the MEMPs do not exhibit TERT activity and/or are negative for the marker CD18.

In a further preferred embodiment the enriched population of the present invention further comprises tissue specific committed cells (TSCCs).

TSCCs are considered to be committed to a particular cell or tissue lineage and are characterised as being Stro-1^{dim} cells. By "committed" we mean that cells are committed to a particular cell or tissue type but need not necessarily be terminally differentiated. A population of cells derived from MPCs expanded in the presence of for example FCS will include TSCCs committed to diverse lineages. Thus a proportion of TSCCs will be committed to say bone, a second proportion of TSCCs will be committed to adipocyte differentiation, and there will also be representative TSCCs of a plurality of different cell or tissue lineages. TSCCs tend to be committed to one cell or tissue lineage type, however they may be bi-potential, that is capable of further differentiation into one of two different cell or tissue types.

Non-limiting examples of the lineages to which TSCCs may be committed include bone precursor cells; hepatocyte progenitors, which are pluripotent for bile duct epithelial cells and hepatocytes; neural restricted cells, which can generate glial cell precursors that progress to oligodendrocytes and astrocytes; neuronal precursors that progress to neurons; precursors for cardiac muscle and cardiomyocytes, glucose-responsive insulin secreting pancreatic beta cell lines. Other TSCCs include but are not limited to chondrocytes, odontoblast, dentin-producing and chondrocytes, and precursor cells of the following: retinal pigment epithelial cells, fibroblasts, skin cells such as keratinocytes, dendritic cells, hair follicle cells, renal duct epithelial cells, smooth and skeletal muscle cells, testicular progenitors, vascular endothelial cells, tendon, ligament, cartilage, adipocyte, fibroblast, marrow stroma, cardiac muscle, smooth muscle, skeletal muscle, pericyte, vascular, epithelial, glial, neuronal, astrocyte and oligodendrocyte cells. TSCCs also include precursor cells that specifically lead to connective tissue including adipose, areolar, osseous, cartilaginous, elastic and fibrous connective tissues.

In one embodiment of the present invention, the enriched cell population comprises TSCCs that are predominantly of one tissue type.

By "predominantly of one tissue type" we mean that at least 20%, more preferably at least 30%, more preferably at least 40%, more preferably at least 50%, more preferably at least 60%, more preferably at least 70%, more preferably at least 80% and more preferably at least 90% of all TSCCs within the population are of the same tissue type.

The MEMPs and TSCCs within the enriched population may be derived from the same individual. Alternatively, the MPC progeny and TSCC may be derived form different individuals (in other words, the MPC progeny and TSCC are allogeneic).

A further finding of the present inventors is that the presence of MPC progeny has a stimulatory effect on proliferation and tissue formation by TSCC. This has been found both *in vitro* and *in vivo.* Thus the invention contemplates a method of stimulating TSCCs proliferation or tissue formation or both by co-culturing with MPC progeny, or by contact with culture supernatant, cell lysates or fractions of cultures of MPC progeny.

The inventors have shown *in vitro,* that proliferation of STRO-1^{dim} cells is enhanced where the proportion of MPCs measured as STRO-1^{bri} cells are kept at a level of 5% or higher. The degree of stimulation is progressively enhanced up to a level where Stro-1^{bri} cells are present up to about 20%. It is envisaged that studies over longer time periods in different culture conditions than those conducted thus far may show that higher concentrations have even greater beneficial effects or that lower levels may also be of benefit. It is proposed therefore that the presence of MPCs at 1, 2, 3 or 4% may also provide a benefit.

The present invention may also provide method of stimulating proliferation of TSCCs by co-culturing TSCCs with MEMPs that have the phenotype Stro-1^{bri}, ALP⁻, or by contacting the TSCCs with culture supernatant, cell lysates or fractions derived from MEMPs that have the phenotype Stro-1^{bri}, ALP⁻.

In a preferred embodiment of this method the MPC progeny are present in the co-culture conditions with TSCC at a level of greater than 1%, more preferably greater than 5%, more preferably greater than 10%, more preferably greater than 20%, more preferably greater than 30%, more preferably greater than 40%, more preferably greater than 50%, more preferably greater than 60%, 70%, 80% or 90%.

This method is equally applicable to those populations of TSCCs that do not normally have MPC progeny present. Thus MPC progeny can be added to the populations of TSCC and maintained in suitable culture conditions for a predetermined time. It is anticipated that numbers of cells can be maintained at an effective level by addition of more MPC progeny from time to time, perhaps with the change of culture media in batch culture, or alternatively every day, or few days in batch or continuous culture systems or may be self sustaining over one, two, three or more passages if present in sufficient numbers initially.

In one embodiment the TSCCs are STRO-1^{dim} cells derived from a purified population of MPCs perhaps using sorting on the basis of STRO-1^{bri} selection or other selection referred to above.

It is proposed that stimulation of TSCCs by MPC progeny is applicable to not only mesenchymal cell types but also others. The data provided to date on RNA and cell surface marker expression suggests that the TSCCs represented in the STRO-1^{dim} population include ectodermal, endodermal, and mesodermal cells or tissues. Cell types that are stimulated by MPC progeny need not necessarily be derived from MPC but may be derived from other sources.

MPC progeny can also be used to stimulate proliferation and/or differentiation of certain haemopoeitic cells. In one embodiment such haemopoietic cells are CD34+ cells.

It is generally contemplated that the invention has applicability to *in vitro* cultivation of cells, that is, in relation to *ex vivo* expanded cultures, however, the invention may also have applicability where the TSCCs are *in situ* in a body tissue site and a population containing MPC progeny are delivered to the site.

Accordingly, in one embodiment of this method the TSCCs are cultured *in vitro.*

In yet another embodiment of this method the TSCCs are positioned at a tissue site of a subject *in vivo,* and the MPC progeny, culture supernatant, cell lysates or fractions of MPC progeny are delivered to the tissue site.

In another embodiment of this method the TSCCs and the MPC progeny are both exogenous and are both delivered to the tissue site.

One such delivery may be adequate, however temporally spaced delivery may provide an accelerated or greater benefit.

In another embodiment the method involves subjecting said cultured population to conditions biasing differentiation of MPC or TSCC to a specific tissue type.

In another embodiment of this method the TSCCs are committed to a tissue type selected from the group consisting of bone, neural tissue, fat, cartilage, skeletal muscle, cardiac muscle, epithelial tissue, osteoblasts, tendon, ligament odontoblast, pericyte, smooth muscle, glial tissue, vascular tissue, endothelial tissue, haematopoietic tissue, hepatic tissue and renal tissue.

In another embodiment of this method the TSCCs are haemopoeitic cells.

In another embodiment the method further comprises subjecting the stimulated TSCC population to conditions biasing differentiation of TSCC to a specific tissue type.

It is envisaged that under appropriate culture conditions the range of cell types that can be generated according to this method include but are not limited to the following, a cartilage tissue cell, a chondrocyte, a hyaline cartilage chondrocyte, a fibrocartilage chondrocyte, an elastic cartilage condrocyte, a ligamentous tissue cell, a fibroblast, a chondrocyte progenitor , a hyaline cartilage chondrocyte progenitor, a fibrocartilage chondrocyte progenitor, an elastic cartilage chondrocyte progenitor, a fibroblast progenitor, a neural tissue cell, a neuron, a glial cell, a progenitor of a neuron, a progenitor of a glial cell, a fat cell, an adipose tissue cell, an adipocyte, a brown adipocyte, a white adipocyte, a progenitor of a white adipocyte, a progenitor of a brown adipocyte, osteoblast, a progenitor of an osteoblast, an odontoblast, a dentin-producing, chondrocyte, an osteocyte, a progenitor of an osteocyte, a bone lining cell, a progenitor of a bone lining cell, a vascular cell, a progenitor of a vascular cell, a tendon cell, a marrow stroma cell, osteoclast- and haemopoietic-supportive stroma cells, a cardiac muscle cell, a progenitor of a cardiac muscle cell, smooth muscle cell, skeletal muscle cell, a pericyte, an endothelial cell, a progenitor of an endothelial cell, an epithelial cell, a progenitor of an epithelial cell, an astrocyte or an oligodendrocyte cell.

The present inventors have also devised culture conditions for increasing the generation of MEMPS. Previous culture conditions do not allow for the preferential expansion of MEMPs. In fact, under previous culture conditions, the proportion of MEMPs typically decreases over time due to their differentiation into Stro-1^{dim} TSCCs.

Accordingly, the present invention also provides a method of enriching for MEMPs that have the phenotype STRO-1^{bri}, ALP⁻, the method comprising culturing STRO-1^{bright} ALP⁺ mesenchymal progenitor cells (MPC) in the presence of one or more stimulatory factors selected from the group consisting of 1α,25-dihydroxyvitamin D₃ (1,25D), platelet derived growth factor (PDGF), tumor necrosis factor α (TNF-α), interleukin -1β (IL-1β) and stromal derived factor 1α (SDF-1α).

The one or more stimulatory factors may include PDGF and/or IL-1β.

In another embodiment of this method of the invention the STRO-1^{bright} ALP⁺ MPC thereof are cultured in the presence of two or more stimulatory factors.

The stimulation of proliferation may be applied to a harvested, unexpanded, population of substantially purified MPCs, comprising at least about 20, 30, 40, 50, 60, 70, 80 or 95% of total cells of the population in which they are present. The effect of stimulating proliferating may be to limit the extent to which MPCs differentiate on *ex vivo* culturing.

In another embodiment of this method of the invention STRO-1^{bright} ALP⁺MPC thereof have been expanded *ex vivo.*

In another embodiment of this method of the invention the STRO-1^{bright} ALP⁺ MPC are an unexpanded population of isolated MPC.

The stimulation may result in an increase in MPC progeny that have the phenotype STRO-1^{bri}, ALP⁻ of more than 10%, preferably more than 20%, preferably more than 40%, preferably more than 50% relative to non stimulated controls.

In another embodiment of this method of the invention the MPC used for culture or expansion are derived from any one or more tissues consisting of the group comprising bone marrow, dental pulp cells, adipose tissue and skin, or perhaps more broadly from adipose tissue, teeth, dental pulp, skin, liver, kidney, heart, retina, brain, hair follicles, intestine, lung, spleen, lymph node, thymus, pancreas, bone, ligament, bone marrow, tendon and skeletal muscle.

In another embodiment of this method of the invention the MPC or progeny thereof are cultured or expanded in the presence of one or more stimulatory factors *in vivo.*

It will be understood from the foregoing that the invention has applicability to *in vitro* proliferation of MPCs however it may equally apply to *in situ* proliferation *in vivo.* Thus the MPC stimulatory factor may be administered directly to a lesion where, for example, it is desirable to stimulate proliferation of resident MPCs, thus the MPC stimulatory factor may be administered alone, or alternatively in combination with a population comprising MPCs. The latter may be viewed as preferable because the numbers of MPCs in tissues is generally very low and additionally it is considered that the beneficial effect to the generation of suitable mesenchymal tissue is likely to be enhanced by the presence of greater numbers of MPCs.

In another embodiment the method further comprises administering exogenous TSCCs.

The present invention may also provide a method of generating a tissue specific committed cell population, the method comprising
culturing a population of cells comprising MPC or progeny thereof and TSCC in the presence of one or more stimulatory factors selected from the group consisting of 1α,25-dihydroxyvitamin D₃ (1,25D), platelet derived growth factor (PDGF), tumor necrosis factor α (TNF-α), interleukin -1β (IL-1β) and stromal derived factor 1α (SDF-1α; and
subjecting said cultured population to conditions biasing differentiation of MPC or TSCC to a specific tissue type.

In one embodiment of this method the tissue type is selected from the group consisting of cardiac muscle, vascular tissue, bone tissue, neural tissue, smooth muscle and endothelial tissue.

The invention will also be understood to encompass a composition comprising MPC progeny and a stimulatory factor. Such a composition is likely to be beneficial therapeutically and thus will be prepared in a pharmaceutically acceptable form. The composition might comprise an enriched or purified population of MPC progeny and one or more stimulatory factors.

The level of the stimulatory factor(s) present in the composition may be determined empirically but in most cases is likely to be in the order of nanograms or tens of nanograms per millilitre.

In the context of *in vivo* delivery it might also be desirable to deliver at the same time in the composition TSCCs. For example, in the case of a lesion in a bone or region thereof, a cardiac muscle or region thereof, a vascular tissue or region thereof or a region comprising one or more endothelial cells the TSCC that is delivered is preferably at least partially committed to a relevant cell type (e.g., an osteoblast, a cardiomyocyte or an endothelial cell). These may be provided as part of a mixed TSCC culture or in a more purified form, for example, being sorted for markers known to be present on the tissue specific committed cell type. Alternatively or additionally the composition being delivered may include one or more differentiation stimulatory factors to differentiate MPCs either present in the composition or present in the target site to one or more tissue types of interest.

Factors that bias differentiation of TSCC or MPC to specific tissue types are described in the Examples provided herein. Conditions that bias differentiation of the MPC or bone precursor cells or bone may involve, for example, culturing in αMEM supplemented with 10% FCS, 100 µM L-ascorbate-2-phosphate, dexamethasone 10⁻⁷ M and 3 mM inorganic phosphate. These conditions have been shown to induce human BM stromal cells to develop a mineralized bone matrix *in vitro* (Gronthos et al., Blood. 84:4164-73, 1994).

Suitable conditions for differentiating the TSCCs into osteoblasts may involve cultivating the TSCCs in the presence of type I collagen, fibrinogen, fibrin, polyglycolic acid, polylactic acid, osteocalcin, or osteonectin. In one particular example, TSCCs are cultivated in the presence of type I collagen, fibrinogen, and fibrin. In an alternative example, TSCCs are cultivated in the presence of type I collagen, fibrinogen, fibrin, osteocalcin, and osteonectin. In the context of this method, type I collagen, fibrinogen, fibrin, polyglycolic acid, polylactic acid, osteocalcin, or osteonectin may be used alone or in the presence of a growth factor. It will be understood that any combination of the compounds listed above in this paragraph is contemplated by the present invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

As will be apparent, preferred features and characteristics of one aspect of the invention are applicable to many other aspects of the invention.

### Production of Genetically Modified Cells

In one embodiment the present invention may provide an isolated genetically modified mesenchymal precursor cell (MPC) progeny having the phenotype STRO-1^{bri}, ALP⁻: Preferably the MEMPs are genetically modified to produce a heterologous protein. Typically, the cells will be genetically modified such that the heterologous protein is secreted from the cells.

Genetically modified cells may be cultured in the presence of at least one cytokine in an amount sufficient to support growth of the modified cells. The genetically modified cells thus obtained may be used immediately (e.g., in transplant), cultured and expanded *in vitro,* or stored for later uses. The modified cells may be stored by methods well known in the art, e.g., frozen in liquid nitrogen.

Genetic modification as used herein encompasses any genetic modification method which involves introduction of an exogenous or foreign polynucleotide into a MEMP or a MEMP precursor (e.g an MPC) or modification of an endogenous gene within a MEMP or MEMP precursor. Genetic modification includes but is not limited to transduction (viral mediated transfer of host DNA from a host or donor to a recipient, either *in vitro* or *in vivo*), transfection (transformation of cells with isolated viral DNA genomes), liposome mediated transfer, electroporation, calcium phosphate transfection or coprecipitation and others. Methods of transduction include direct co-culture of cells with producer cells (Bregni et al., Blood 80:1418-1422, 1992) or culturing with viral supernatant alone with or without appropriate growth factors and polycations (Xu et al., Exp. Hemat. 22:223-230, 1994).

A polynucleotide encoding a heterologous polypeptide is preferably introduced to a host cell in a vector. The vector preferably includes the necessary elements for the transcription and translation of the inserted coding sequence. Methods used to construct such vectors are well known in the art. For example, techniques for constructing suitable expression vectors are described in detail in Sambrook et al., Molecular Cloning: A Laboratory Manual, Cold Spring Harbor Press, N.Y. (3rd Ed., 2000); and Ausubel et al., Current Protocols in Molecular Biology, John Wiley & Sons, Inc., New York (1999).

Vectors may include but are not limited to viral vectors, such as retroviruses, adenoviruses, adeno-associated viruses, and herpes simplex viruses; cosmids; plasmid vectors; synthetic vectors; and other recombination vehicles typically used in the art. Vectors containing both a promoter and a cloning site into which a polynucleotide can be operatively linked are well known in the art. Such vectors are capable of transcribing RNA *in vitro* or *in vivo,* and are commercially available from sources such as Stratagene (La Jolla, Calif.) and Promega Biotech (Madison, Wis.). Specific examples include, pSG, pSV2CAT, pXtl from Stratagene; and pMSG, pSVL, pBPV and pSVK3 from Pharmacia.

Preferred vectors include retroviral vectors (see, Coffin et al., "Retroviruses", Chapter 9 pp; 437-473, Cold Springs Harbor Laboratory Press, 1997). Vectors useful in the invention can be produced recombinantly by procedures well known in the art. For example, WO94/29438, WO97/21824 and WO97/21825 describe the construction of retroviral packaging plasmids and packing cell lines. Exemplary vectors include the pCMV mammalian expression vectors, such as pCMV6b and pCMV6c (Chiron Corp.), pSFFV-Neo, and pBluescript-Sk+. Non-limiting examples of useful retroviral vectors are those derived from murine, avian or primate retroviruses. Common retroviral vectors include those based on the Moloney murine leukemia virus (MoMLV-vector). Other MoMLV derived vectors include, Lmily, LINGFER, MINGFR and MINT (Chang et al., Blood 92:1-11, 1998). Additional vectors include those based on Gibbon ape leukemia virus (GALV) and Moloney murine sarcoma virus (MOMSV) and spleen focus forming virus (SFFV). Vectors derived from the murine stem cell virus (MESV) include MESV-MiLy (Agarwal et al., J. of Virology, 72:3720-3728, 1998). Retroviral vectors also include vectors based on lentiviruses, and non-limiting examples include vectors based on human immunodeficiency virus (HIV-1 and HIV-2).

In producing retroviral vector constructs, the viral gag, pol and env sequences can be removed from the virus, creating room for insertion of foreign DNA sequences. Genes encoded by foreign DNA are usually expressed under the control a strong viral promoter in the long terminal repeat (LTR). Selection of appropriate control regulatory sequences is dependent on the host cell used and selection is within the skill of one in the art. Numerous promoters are known in addition to the promoter of the LTR. Non-limiting examples include the phage lambda PL promoter, the human cytomegalovirus (CMV) immediate early promoter; the U3 region promoter of the Moloney Murine Sarcoma Virus (MMSV), Rous Sacroma Virus (RSV), or Spleen Focus Forming Virus (SFFV); Granzyme A promoter; and the Granzyme B promoter. Additionally inducible or multiple control elements may be used. The selection of a suitable promoter will be apparent to those skilled in the art.

Such a construct can be packed into viral particles efficiently if the gag, pol and env functions are provided in trans by a packing cell line. Therefore, when the vector construct is introduced into the packaging cell, the gag-pol and env proteins produced by the cell, assemble with the vector RNA to produce infectious virons that are secreted into the culture medium. The virus thus produced can infect and integrate into the DNA of the target cell, but does not produce infectious viral particles since it is lacking essential packaging sequences. Most of the packing cell lines currently in use have been transfected with separate plasmids, each containing one of the necessary coding sequences, so that multiple recombination events are necessary before a replication competent virus can be produced. Alternatively the packaging cell line harbours a provirus. The provirus has been crippled so that although it may produce all the proteins required to assemble infectious viruses, its own RNA cannot be packaged into virus. RNA produced from the recombinant virus is packaged instead. Therefore, the virus stock released from the packaging cells contains only recombinant virus. Non-limiting examples of retroviral packaging lines include PA12, PA317, PE501, PG13, PSI.CRIP, RDI 14, GP7C-tTA-G10, ProPak-A (PPA-6), and PT67. Reference is made to Miller et al., Mol. Cell Biol. 6:2895, 1986; Miller et al., Biotechniques 7:980, 1989; Danos et al., Proc. Natl. Acad. Sci. USA 85:6460, 1988; Pear et al., Proc. Natl. Acad. Sci. USA 90:8392-8396, 1993; and Finer et al., Blood 83:43-50, 1994.

Other suitable vectors include adenoviral vectors (see, Frey et al., Blood 91:2781, 1998; and WO 95/27071) and adeno-associated viral vectors. These vectors are all well known in the art, e.g., as described in Chatterjee et al., Current Topics in Microbiol. And Immunol., 218:61-73, 1996; Stem cell Biology and Gene Therapy, eds. Quesenberry et al., John Wiley & Sons, 1998; and U.S. Pat. Nos. 5,693,531 and 5,691,176. The use of adenovirus-derived vectors may be advantageous under certain situation because they are not capable of infecting non-dividing cells. Unlike retroviral DNA, the adenoviral DNA is not integrated into the genome of the target cell. Further, the capacity to carry foreign DNA is much larger in adenoviral vectors than retroviral vectors. The adeno-associated viral vectors are another useful delivery system. The DNA of this virus may be integrated into non-dividing cells, and a number of polynucleotides have been successful introduced into different cell types using adeno-associated viral vectors.

In some embodiments, the construct or vector will include two or more heterologous polynucleotide sequences. Preferably the additional nucleic acid sequence is a polynucleotide which encodes a selective marker, a structural gene, a therapeutic gene, or a cytokine/chemokine gene.

A selective marker may be included in the construct or vector for the purposes of monitoring successful genetic modification and for selection of cells into which DNA has been integrated. Non-limiting examples include drug resistance markers, such as G148 or hygromycin. Additionally negative selection may be used, for example wherein the marker is the HSV-tk gene. This gene will make the cells sensitive to agents such as acyclovir and gancyclovir. The NeoR (neomycin/G148 resistance) gene is commonly used but any convenient marker gene may be used whose gene sequences are not already present in the target cell can be used. Further non-limiting examples include low-affinity Nerve Growth Factor (NGFR), enhanced fluorescent green protein (EFGP), dihydrofolate reductase gene (DHFR) the bacterial hisD gene, murine CD24 (HSA), murine CD8a(lyt), bacterial genes which confer resistance to puromycin or phleomycin, and β-glactosidase.

The additional polynucleotide sequence(s) may be introduced into the host cell on the same vector as the polynucleotide sequence encoding the heterologous protein, or the additional polynucleotide sequence may be introduced into the host cells on a second vector. In a preferred embodiment, a selective marker will be included on the same vector as the polynucleotide encoding the heterologous protein.

The present invention also encompasses genetically modifying the promoter region of an endogenous gene such that expression of the endogenous gene is up-regulated resulting in the increased production of the encoded protein compared to a wild type MEMPs.

### Administration of Stimulatory Factors

Methods of the present invention may involve administration of one or more stimulatory factors to a subject in order to enrich for MEMPs *in situ.*

These methods may involve administering one or more stimulatory factors such as 1α,25-dihydroxyvitamin D₃ (1,25D), platelet derived growth factor (PDGF), tumor necrosis factor α (TNF- α), interleukin -1β (IL-1β) and stromal derived factor 1α (SDF-1α) topically, systematically, or locally such as within an implant or device.

In one particular embodiment the invention provides a method of enriching for MEMPs in a subject in need thereof by administering a stimulatory factor systemically to the subject. For example, the stimulatory factor may be administered by subcutaneous or intramuscular injection.

This embodiment of the invention may be useful for the treatment of systemic degenerative diseases where enrichment of MEMPs in particular tissues is desirable. Examples of systemic degenerative diseases that can be treated in this way include osteoporosis or fractures, degenerative diseases of cartilage, atherosclerosis, peripheral artery diseases or cardiovascular diseases and the like.

Thus, according to the present invention, stimulatory factors in a therapeutically or prophylactically effective amount may be used in treating diseases or disorders selected from the group consisting of autoimmune diseases, acute chronic inflammation, cancer, cardiovascular disease, infectious disease, and inflammatory disorders including rheumatoid arthritis, chronic inflammatory bowel disease, chronic inflammatory pelvic disease, multiple sclerosis, asthma, osteoarthritis, atherosclerosis, psoriasis, rhinitis, autoimmunity, and organ transplant rejection. In one example, such compositions include one or more stimulatory factors in a therapeutically or prophylactically effective amount sufficient to be used to assist in stimulating the production of tissue specific cells.

A "therapeutically effective amount" refers to an amount effective, at dosages and for periods of time necessary, to achieve enrichment of MEMPs.

A "prophylactically effective amount" refers to an amount effective, at dosages and for periods of time necessary, to achieve the desired prophylactic result, such as preventing or inhibiting death of MPC or progeny derived therefrom.

In particular embodiments, a preferred range for stimulatory factors may be 0.1 nM-0.1 M, 0.1 nM-0.05 M, 0.05 nM-15 µM or 0.01 nM-10 µM. It is to be noted that dosage values may vary with the severity of the condition to be alleviated. For any particular subject, specific dosage regimens may be adjusted over time according to the individual need and the professional judgement of the person administering or supervising the administration of the compositions. Dosage ranges set forth herein are exemplary only and do not limit the dosage ranges that may be selected by medical practitioners.

The amount of stimulatory factor in the composition may vary according to factors such as the disease state, age, sex, and weight of the individual. Dosage regimens may be adjusted to provide the optimum therapeutic response. For example, a single bolus may be administered, several divided doses may be administered over time or the dose may be proportionally reduced or increased as indicated by the exigencies of the therapeutic situation. It may be advantageous to formulate parenteral compositions in dosage unit form for ease of administration and uniformity of dosage. "Dosage unit form" as used herein refers to physically discrete units suited as unitary dosages for subjects to be treated; each unit containing a predetermined quantity of active compound calculated to produce the desired therapeutic effect in association with the required pharmaceutical carrier.

It will be appreciated that the stimulatory factor may be administered in the form of a composition comprising a pharmaceutically acceptable carrier or excipient.

As used herein "pharmaceutically acceptable carrier" or "excipient" includes any and all solvents, dispersion media, coatings, antibacterial and antifungal agents, isotonic and absorption delaying agents, and the like that are physiologically compatible. In one embodiment, the carrier is suitable for parenteral administration. Alternatively, the carrier can be suitable for intravenous, intraperitoneal, intramuscular, sublingual or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

Pharmaceutical formulations for parenteral administration may include liposomes. Liposomes and emulsions are well known examples of delivery vehicles or carriers that are especially useful for hydrophobic drugs. Depending on biological stability of the therapeutic reagent, additional strategies for protein stabilization may be employed. Furthermore, one may administer the drug in a targeted drug delivery system, for example, in a liposome coated with target-specific antibody. The liposomes will bind to the target protein and be taken up selectively by the cell expressing the target protein.

Therapeutic compositions typically should be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin. Moreover, the stimulatory factor may be administered in a time release formulation, for example in a composition which includes a slow release polymer. The active compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are patented or generally known to those skilled in the art.

Additionally, suspensions of stimulatory factors may be prepared as appropriate oily suspensions for injection. Suitable lipophilic solvents or vehicles include fatty oils such as sesame oil; or synthetic fatty acid esters, such as ethyl oleate or triglycerides; or liposomes. Suspensions to be used for injection may also contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof. In accordance with an alternative aspect of the invention, the stimulatory factor may be formulated with one or more additional compounds that enhance its solubility.

If the stimulatory compounds are to be administered by inhalation, they may be conveniently delivered in the form of an aerosol spray presentation from pressurized packs or a nebuliser; together with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of gelatin, for example, for use in an inhaler may be formulated containing a powder mix of the compound and a suitable powder base such as starch or lactose.

### Administration of cellular compositions of the present invention

The cellular compositions of the present invention comprising MEMPs and/or TSCCs may be useful for the regeneration of tissue of various types, including bone, cartilage, tendon, ligament, muscle, skin, and other connective tissue, as well as nerve, cardiac, liver, lung, kidney, pancreas, brain, and other organ tissues.

In some embodiments, the compositions of the present invention may be administered in combination with an appropriate matrix, for instance, for supporting the MEMPs and providing a surface for bone, cartilage, muscle, nerve, epidermis and/or other connective tissue growth. The matrix may be in the form of traditional matrix biomaterials. The matrix may provide slow release of the expressed protein and differentiated cells and/or the appropriate environment for presentation thereof. In some embodiments, various collagenous and non-collagenous proteins are expected to be upregulated and secreted from the MEMPs. This phenomenon accelerates tissue regeneration by enhancing matrix deposition. Matrix proteins can also be expressed in the genetically engineered cells and enhance the engraftment and attachment of transplanted cells into the transplant area.

The choice of matrix material is based on biocompatibility, biodegradability, mechanical properties, cosmetic appearance and interface properties. The particular application of the cellular based compositions will define the appropriate formulation. Potential matrices for the compositions may be biodegradable and chemically defined calcium sulfate, tricalcium phosphate, hydroxyapatite, polylactic acid and polyanhydrides. Other potential materials are biodegradable and biologically well defined, such as bone or dermal collagen. Further matrices are comprised of pure proteins or extracellular matrix components. Other potential matrices are nonbiodegradable and chemically defined, such as sintered hydroxyapatite, bioglass, aluminates, or other ceramics. Matrices may be comprised of combinations of any of the above mentioned types of material, such as polylactic acid and hydroxyapatite or collagen and tricalcium phosphate. The bioceramics may be altered in composition, such as in calcium-aluminate-phosphate and processing to alter pore size, particle size, particle shape, and biodegradability.

The cellular compositions of the invention may be used to treat patients requiring the repair or replacement of cartilage or bone tissue resulting from disease or trauma or failure of the tissue to develop normally, or to provide a cosmetic function, such as to augment facial or other features of the body. Treatment may entail the use of the cells of the invention to produce new cartilage tissue or bone tissue. For example, compositions comprising undifferentiated or chondrogenic differentiation-induced precursor cells may be used to treat a cartilage condition, for example, rheumatoid arthritis or osteoarthritis or a traumatic or surgical injury to cartilage. As another example, compositions comprising bone precursor cells may be used to treat bone conditions, including metabolic and non-metabolic bone diseases. Examples of bone conditions include meniscal tears, spinal fusion, spinal disc removal, spinal reconstruction, bone fractures, bone/spinal deformation, osteosarcoma, myeloma, bone dysplasia, scoliosis, osteoporosis, periodontal disease, dental bone loss, osteomalacia, rickets, fibrous osteitis, renal bone dystrophy, and Paget's disease of bone.

The cellular compositions of the invention may be administered alone or as admixtures with other cells. Cells that may be administered in conjunction with the compositions of the present invention include, but are not limited to, other multipotent or pluripotent cells or chondrocytes, chondroblasts, osteocytes, osteoblasts, osteoclasts, bone lining cells, stem cells, or bone marrow cells. The cells of different types may be admixed with a composition of the invention immediately or shortly prior to administration, or they may be co-cultured together for a period of time prior to administration.

The cellular compositions of the invention may be administered with other beneficial drugs or biological molecules (growth factors, trophic factors). When the MEMPs are administered with other agents, they may be administered together in a single pharmaceutical composition, or in separate pharmaceutical compositions, simultaneously or sequentially with the other agents (either before or after administration of the other agents). Bioactive factors which may be co-administered include anti-apoptotic agents (e.g., EPO, EPO mimetibody, TPO, IGF-I and IGF-II, HGF, caspase inhibitors); anti-inflammatory agents (e.g., p38 MAPK inhibitors, TGF-beta inhibitors, statins, IL-6 and IL-1 inhibitors, PEMIROLAST, TRANILAST, REMICADE, SIROLIMUS, and NSAIDs (non-steroidal anti-inflammatory drugs; e.g., TEPOXALIN, TOLMETIN, SUPROFEN); immunosupressive/immunomodulatory agents (e.g., calcineurin inhibitors, such as cyclosporine, tacrolimus; mTOR inhibitors (e.g., SIROLIMUS, EVEROLIMUS); anti-proliferatives (e.g., azathioprine, mycophenolate mofetil); corticosteroids (e.g., prednisolone, hydrocortisone); antibodies such as monoclonal anti-IL-2Ralpha receptor antibodies (e.g., basiliximab, daclizumab), polyclonal anti-T-cell antibodies (e.g., anti-thymocyte globulin (ATG); anti-lymphocyte globulin (ALG); monoclonal anti-T cell antibody OKT3)); anti-thrombogenic agents (e.g., heparin, heparin derivatives, urokinase, PPack (dextrophenylalanine proline arginine chloromethylketone), antithrombin compounds, platelet receptor antagonists, anti-thrombin antibodies, anti-platelet receptor antibodies, aspirin, dipyridamole, protamine, hirudin, prostaglandin inhibitors, and platelet inhibitors); and anti-oxidants (e.g., probucol, vitamin A, ascorbic acid, tocopherol, coenzyme Q-10, glutathione, L-cysteine, N-acetylcysteine) as well as local anesthetics. As another example, the cells may be co-administered with scar inhibitory factor as described in U.S. Pat. No. 5,827,735.

In one embodiment, cellular compositions of the invention are administered as undifferentiated cells, i.e., as cultured in Growth Medium. Alternatively, the cellular compositions may be administered following exposure in culture to conditions that stimulate differentiation toward a desired phenotype, for example, an osteogenic phenotype.

The cellular compositions of the invention may be surgically implanted, injected, delivered (e.g., by way of a catheter or syringe), or otherwise administered directly or indirectly to the site in need of repair or augmentation. The cells may be administered by way of a matrix (e.g., a three-dimensional scaffold). The cells may be administered with conventional pharmaceutically acceptable carriers. Routes of administration of the cells of the invention or compositions or components (e.g., ECM, cell lysate, conditioned medium) thereof include intramuscular, ophthalmic, parenteral (including intravenous), intraarterial, subcutaneous, oral, and nasal administration. Particular routes of parenteral administration include, but are not limited to, intramuscular, subcutaneous, intraperitoneal, intracerebral, intraventricular, intracerebroventricular, intrathecal, intracisternal, intraspinal and/or peri-spinal routes of administration.

When cells are administered in semi-solid or solid devices, surgical implantation into a precise location in the body is typically a suitable means of administration. Liquid or fluid pharmaceutical compositions, however, may be administered to a more general location (e.g., throughout a diffusely affected area, for example), from which they migrate to a particular location, e.g., by responding to chemical signals.

Other embodiments encompass methods of treatment by administering pharmaceutical compositions comprising cellular components (e.g., cell lysates or components thereof) or products (e.g., extracellular matrix, trophic and other biological factors produced through genetic modification).

Dosage forms and regimes for administering cellular compositions described herein are developed in accordance with good medical practice, taking into account the condition of the individual patient, e.g., nature and extent of the condition being treated, age, sex, body weight and general medical condition, and other factors known to medical practitioners. Thus, the effective amount of a pharmaceutical composition to be administered to a patient is determined by these considerations as known in the art.

In some embodiments of the invention, it may not be necessary or desirable to immunosuppress a patient prior to initiation of therapy with cellular compositions of the present invention. Accordingly, transplantation with allogeneic, or even xenogeneic, MEMPs may be tolerated in some instances.

However, in other instances it may be desirable or appropriate to pharmacologically immunosuppress a patient prior to initiating cell therapy. This may be accomplished through the use of systemic or local immunosuppressive agents, or it may be accomplished by delivering the cells in an encapsulated device. MEMPs may be encapsulated in a capsule that is permeable to nutrients and oxygen required by the cell and therapeutic factors the cell is yet impermeable to immune humoral factors and cells. Preferably the encapsulant is hypoallergenic, is easily and stably situated in a target tissue, and provides added protection to the implanted structure. These and other means for reducing or eliminating an immune response to the transplanted cells are known in the art. As an alternative, MEMPs may be genetically modified to reduce their immunogenicity.

Survival of transplanted MEMPs in a living patient can be determined through the use of a variety of scanning techniques, e.g., computerized axial tomography (CAT or CT) scan, magnetic resonance imaging (MRI) or positron emission tomography (PET) scans. Determination of transplant survival can also be done post mortem by removing the target tissue, and examining it visually or through a microscope. Alternatively, cells can be treated with stains that are specific for cells of a specific lineage. Transplanted cells can also be identified by prior incorporation of tracer dyes such as rhodamine- or fluorescein-labeled microspheres, fast blue, bisbenzamide, ferric microparticles, or genetically introduced reporter gene products, such as beta-galactosidase or beta-glucuronidase.

Functional integration of transplanted MEMPs into a subject can be assessed by examining restoration of the function that was damaged or diseased, for example, restoration of joint or bone function, or augmentation of function.

Cellular compositions of the invention may include one or more bioactive factors, for example but not limited to a growth factor, a differentiation-inducing factor, a cell survival factor such as caspase inhibitor, an anti-inflammatory agent such as p38 kinase inhibitor, or an angiogenic factor such as VEGF or bFGF. Some examples of bioactive factors include PDGF-bb, EGF, bFGF, IGF-1, and LIF.

Alternatively, MEMPs to be transplanted may be genetically engineered to express such growth factors, antioxidants, antiapoptotic agents, anti-inflammatory agents, or angiogenic factors.

Pharmaceutical compositions of the invention may comprise homogeneous or heterogeneous populations of MEMPs, extracellular matrix or cell lysate thereof, or conditioned medium thereof in a pharmaceutically acceptable carrier. Pharmaceutically acceptable carriers for the cells of the invention include organic or inorganic carrier substances suitable which do not deleteriously react with the cells of the invention or compositions or components thereof. To the extent they are biocompatible, suitable pharmaceutically acceptable carriers include water, salt solution (such as Ringer's solution), alcohols, oils, gelatins, and carbohydrates, such as lactose, amylose, or starch, fatty acid esters, hydroxymethylcellulose, and polyvinyl pyrolidine. Such preparations can be sterilized, and if desired, mixed with auxiliary agents such as lubricants, preservatives, stabilizers, wetting agents, emulsifiers, salts for influencing osmotic pressure, buffers, and coloring. Pharmaceutical carriers suitable for use in the present invention are known in the art and are described, for example, in Pharmaceutical Sciences (17.sup.th Ed., Mack Pub. Co., Easton, Pa.) and WO 96/05309.

One or more other components may be added to transplanted cells, including selected extracellular matrix components, such as one or more types of collagen known in the art, and/or growth factors, platelet-rich plasma, and drugs. Alternatively, the cells of the invention may be genetically engineered to express and produce for growth factors. Details on genetic engineering of the cells of the invention are provided herein.

In a non-limiting embodiment, a formulation comprising the cells of the invention is prepared for administration directly to the site where the production of new tissue, such as bone tissue, is desired. For example, and not by way of limitation, the MEMPs may be suspended in a hydrogel solution for injection. Examples of suitable hydrogels for use in the invention include self-assembling peptides, such as RAD16. Alternatively, the hydrogel solution containing the cells may be allowed to harden, for instance in a mold, to form a matrix having cells dispersed therein prior to implantation. Or, once the matrix has hardened, the cell formations may be cultured so that the cells are mitotically expanded prior to implantation. The hydrogel is an organic polymer (natural or synthetic) which is cross-linked via covalent, ionic, or hydrogen bonds to create a three-dimensional open-lattice structure which entraps water molecules to form a gel. Examples of materials which can be used to form a hydrogel include polysaccharides such as alginate and salts thereof, peptides, polyphosphazines, and polyacrylates, which are cross-linked ionically, or block polymers such as polyethylene oxide-polypropylene glycol block copolymers which are crosslinked by temperature or pH, respectively. In some embodiments, the support for the MPC or progeny derived therefrom is biodegradable.

In some embodiments of the invention, the formulation comprises an *in situ* polymerizable gel, as described, for example, in U.S. Patent Application Publication 2002/0022676; Anseth et al., J. Control Release, 78(1-3): 199-209 (2002); Wang et al., Biomaterials, 24(22):3969-80 (2003).

In some embodiments, the polymers are at least partially soluble in aqueous solutions, such as water, buffered salt solutions, or aqueous alcohol solutions, that have charged side groups, or a monovalent ionic salt thereof. Examples of polymers with acidic side groups that can be reacted with cations are poly(phosphazenes), poly(acrylic acids), poly(methacrylic acids), copolymers of acrylic acid and methacrylic acid, poly(vinyl acetate), and sulfonated polymers, such as sulfonated polystyrene. Copolymers having acidic side groups formed by reaction of acrylic or methacrylic acid and vinyl ether monomers or polymers can also be used. Examples of acidic groups are carboxylic acid groups, sulfonic acid groups, halogenated (preferably fluorinated) alcohol groups, phenolic OH groups, and acidic OH groups.

Examples of polymers with basic side groups that can be reacted with anions are poly(vinyl amines), poly(vinyl pyridine), poly(vinyl imidazole), and some imino substituted polyphosphazenes. The ammonium or quaternary salt of the polymers can also be formed from the backbone nitrogens or pendant imino groups. Examples of basic side groups are amino and imino groups.

Alginate can be ionically cross-linked with divalent cations, in water, at room temperature, to form a hydrogel matrix. Due to these mild conditions, alginate has been the most commonly used polymer for hybridoma cell encapsulation, as described, for example, in U.S. Pat. No. 4,352,883 to Lim. In the Lim process, an aqueous solution containing the biological materials to be encapsulated is suspended in a solution of a water soluble polymer, the suspension is formed into droplets which are configured into discrete microcapsules by contact with multivalent cations, then the surface of the microcapsules is crosslinked with polyamino acids to form a semipermeable membrane around the encapsulated materials.

Polyphosphazenes are polymers with backbones consisting of nitrogen and phosphorous separated by alternating single and double bonds. Each phosphorous atom is covalently bonded to two side chains.

The polyphosphazenes suitable for cross-linking have a majority of side chain groups which are acidic and capable of forming salt bridges with di- or trivalent cations. Examples of preferred acidic side groups are carboxylic acid groups and sulfonic acid groups. Hydrolytically stable polyphosphazenes are formed of monomers having carboxylic acid side groups that are crosslinked by divalent or trivalent cations such as Ca²⁺ or Al³⁺. Polymers can be synthesized that degrade by hydrolysis by incorporating monomers having imidazole, amino acid ester, or glycerol side groups. For example, a polyanionic poly[bis(carboxylatophenoxy)]phosphazene (PCPP) can be synthesized, which is cross-linked with dissolved multivalent cations in aqueous media at room temperature or below to form hydrogel matrices.

Biodegradable polyphosphazenes have at least two differing types of side chains, acidic side groups capable of forming salt bridges with multivalent cations, and side groups that hydrolyze under *in vivo* conditions, e.g., imidazole groups, amino acid esters, glycerol and glucosyl.

Hydrolysis of the side chain results in erosion of the polymer. Examples of hydrolyzing side chains are unsubstituted and substituted imidizoles and amino acid esters in which the group is bonded to the phosphorous atom through an amino linkage (polyphosphazene polymers in which both R groups are attached in this manner are known as polyaminophosphazenes). For polyimidazolephosphazenes, some of the "R" groups on the polyphosphazene backbone are imidazole rings, attached to phosphorous in the backbone through a ring nitrogen atom. Other "R" groups can be organic residues that do not participate in hydrolysis, such as methyl phenoxy groups or other groups shown in the scientific paper of Allcock et al., Macromolecule 10:824 (1977). Methods of synthesis of the hydrogel materials, as well as methods for preparing such hydrogels, are known in the art.

Other components may also be included in the formulation, including but not limited to any of the following: (1) buffers to provide appropriate pH and isotonicity; (2) lubricants; (3) viscous materials to retain the cells at or near the site of administration, including, for example, alginates, agars and plant gums; and (4) other cell types that may produce a desired effect at the site of administration, such as, for example, enhancement or modification of the formation of tissue or its physicochemical characteristics, or as support for the viability of the cells, or inhibition of inflammation or rejection. The cells may be covered by an appropriate wound covering to prevent cells from leaving the site. Such wound coverings are known as those of skill in the art.

### Formulation of a Bone Tissue Patch

Culture or co-cultures of MEMPs in a pre-shaped well enables the manufacture of a tissue patch of pre-determined thickness and volume. The volume of the resulting tissue patch is dependent upon the volume of the well and upon the number of MEMPs in the well. Tissue of optimal pre-determined volume may be prepared by routine experimentation by altering either or both of the aforementioned parameters.

The cell contacting surface of the well may be coated with a molecule that discourages adhesion of MEMPs to the cell contacting surface. Preferred coating reagents include silicon based reagents i.e., dichlorodimethylsilane or polytetrafluoroethylene based reagents, i.e., TEFLON. Procedures for coating materials with silicon based reagents, specifically dichlorodimethylsilane, are well known in the art. See for example, Sambrook et al. (1989) "Molecular Cloning A Laboratory Manual", Cold Spring Harbor Laboratory Press. It is appreciated that other biocompatible reagents that prevent the attachment of cells to the surface of the well may be useful in the practice of the instant invention.

Alternatively, the well may be cast from a pliable or moldable biocompatible material that does not permit attachment of cells per se. Preferred materials that prevent such cell attachment include, but are not limited to, agarose, glass, untreated cell culture plastic and polytetrafluoroethylene, i.e., TEFLON. Untreated cell culture plastics, i.e., plastics that have not been treated with or made from materials that have an electrostatic charge are commercially available, and may be purchased, for example, from Falcon Labware, Becton-Dickinson, Lincoln Park, N.J. The aforementioned materials, however, are not meant to be limiting. It is appreciated that any other pliable or moldable biocompatible material that inherently discourages the attachment of MEMPs may be useful in the practice of the instant invention.

MEMPs in suspension may be seeded into and cultured in the pre-shaped well. The MEMPs may be induced to differentiate to a chondrogenic or osteogenic phenotype in culture in the well or may have been induced to differentiate prior to seeding in the well. The cells may be diluted by the addition of culture medium to a cell density of about 1 x 10⁵ to 1 x 10⁹ cells per milliliter.

The cells may form a cohesive plug of cells. The cohesive plug of cells may be removed from the well and surgically implanted into the tissue defect. It is anticipated that undifferentiated MPC or progeny derived therefrom may differentiate *in situ* thereby to form tissue *in vivo.*

Bone defects may be identified inferentially by using computer aided tomography (CAT scanning); X-ray examination, magnetic resonance imaging (MRI), analysis of synovial fluid or serum markers or by any other procedures known in the art. Defects in mammals also are readily identifiable visually during arthroscopic examination or during open surgery of the joint. Treatment of the defects can be effected during an arthroscopic or open surgical procedure using the methods and compositions disclosed herein.

Accordingly, once the defect has been identified, the defect may be treated by the following steps of (1) surgically implanting at the pre-determined site a tissue patch prepared by the methodologies described herein, and (2) permitting the tissue patch to integrate into pre-determined site.

The tissue patch optimally has a size and shape such that when the patch is implanted into the defect, the edges of the implanted tissue contact directly the edges of the defect. In addition, the tissue patch may be fixed in place during the surgical procedure. This can be effected by surgically fixing the patch into the defect with biodegradable sutures and/or by applying a bioadhesive to the region interfacing the patch and the defect.

In some instances, damaged tissue may be surgically excised prior to the implantation of the patch of tissue.

### Transplantation of MEMPs using scaffolds

The cellular compositions of the invention or co-cultures thereof may be seeded onto or into a three-dimensional scaffold and implanted *in vivo,* where the seeded cells will proliferate on the framework and form a replacement tissue, such as bone tissue, *in vivo* in cooperation with the cells of the patient.

For example, but not by way of limitation, the scaffold may be designed such that the scaffold structure: (1) supports the seeded cells without subsequent degradation; (2) supports the cells from the time of seeding until the tissue transplant is remodeled by the host tissue; (2) allows the seeded cells to attach, proliferate, and develop into a tissue structure having sufficient mechanical integrity to support itself *in vitro,* at which point, the scaffold is degraded. A review of scaffold design is provided by Hutmacher, J. Biomat. Sci. Polymer Edn., 12(1):107-124 (2001).

Scaffolds of the invention can be administered in combination with any one or more growth factors, cells, for example stem cells, bone marrow cells, chondrocytes, chondroblasts, osteocytes, osteoblasts, osteoclasts, bone lining cells, or their precursors, drugs or other components described above that stimulate tissue formation or otherwise enhance or improve the practice of the invention. The MEMPs to be seeded onto the scaffolds may be genetically engineered to express growth factors or drugs.

The cells of the invention can be used to produce new tissue *in vitro,* which can then be implanted, transplanted or otherwise inserted into a site requiring tissue repair, replacement or augmentation in a patient.

In a non-limiting embodiment, the cells of the invention are used to produce a three-dimensional tissue construct *in vitro,* which is then implanted *in vivo.* As an example of the production of three-dimensional tissue constructs, see U.S. Pat. No. 4,963,489. For example, the cells of the invention may be inoculated or "seeded" onto a three-dimensional framework or scaffold, and proliferated or grown *in vitro* to form a living tissue that can be implanted *in vivo.*

The cells of the invention can be grown freely in a culture vessel to sub-confluency or confluency, lifted from the culture and inoculated onto a three-dimensional framework. Inoculation of the three-dimensional framework with a high concentration of cells, e.g., approximately 10⁶ to 5 x 10⁷ cells per milliliter, will result in the establishment of the three-dimensional support in relatively shorter periods of time.

Examples of scaffolds which may be used in the present invention include nonwoven mats, porous foams, or self assembling peptides. Nonwoven mats may, for example, be formed using fibers comprised of a synthetic absorbable copolymer of glycolic and lactic acids (PGA/PLA), sold under the tradename VICRYL (Ethicon, Inc., Somerville, N.J.). Foams, composed of, for example, poly(epsilon-caprolactone)/poly(glycolic acid) (PCL/PGA) copolymer, formed by processes such as freeze-drying, or lyophilized, as discussed in U.S. Pat. No. 6,355,699, are also possible scaffolds. Hydrogels such as self-assembling peptides (e.g., RAD16) may also be used. These materials are frequently used as supports for growth of tissue.

The three-dimensional framework may be made of ceramic materials including, but not limited to: mono-, di-, tri-, alpha-tri-, beta-tri-, and tetra-calcium phosphate, hydroxyapatite, fluoroapatites, calcium sulfates, calcium fluorides, calcium oxides, calcium carbonates, magnesium calcium phosphates, biologically active glasses such as BIOGLASS (University of Florida, Gainesville, Fla.), and mixtures thereof. There are a number of suitable porous biocompatible ceramic materials currently available on the commercial market such as SURGIBON (Unilab Surgibone, Inc., Canada), ENDOBON (Merck Biomaterial France, France), CEROS (Mathys, A. G., Bettlach, Switzerland), and INTERPORE (Interpore, Irvine, Calif., United States), and mineralized collagen bone grafting products such as HEALOS (Orquest, Inc., Mountain View, Calif.) and VITOSS, RHAKOSS, and CORTOSS (Orthovita, Malvern, Pa.). The framework may be a mixture, blend or composite of natural and/or synthetic materials. In some embodiments, the scaffold is in the form of a cage. In a preferred embodiment, the scaffold is coated with collagen.

According to a preferred embodiment, the framework is a felt, which can be composed of a multifilament yarn made from a bioabsorbable material, e.g., PGA, PLA, PCL copolymers or blends, or hyaluronic acid. The yarn is made into a felt using standard textile processing techniques consisting of crimping, cutting, carding and needling.

In another preferred embodiment the cells of the invention are seeded onto foam scaffolds that may be composite structures. In addition, the three-dimensional framework may be molded into a useful shape, such as that of the external portion of the ear, a bone, joint or other specific structure in the body to be repaired, replaced or augmented.

In another preferred embodiment, the cells are seeded onto a framework comprising a prosthetic device for implantation into a patient, as described in U.S. Pat. No. 6,200,606. As described therein, a variety of clinically useful prosthetic devices have been developed for use in bone and cartilage grafting procedures. (see e.g. Bone Grafts and Bone Substitutions. Ed. M. B. Habal & A. H. Reddi, W. B. Saunders Co., 1992). For example, effective knee and hip replacement devices have been and continue to be widely used in the clinical environment. Many of these devices are fabricated using a variety of inorganic materials having low immunogenic activity, which safely function in the body. Examples of synthetic materials which have been tried and proven include titanium alloys, calcium phosphate, ceramic hydroxyapatite, and a variety of stainless steel and cobalt-chrome alloys. These materials provide structural support and can form a scaffolding into which host vascularization and cell migration can occur.

The framework may be treated prior to inoculation of the cells of the invention in order to enhance cell attachment. For example, prior to inoculation with the cells of the invention, nylon matrices could be treated with 0.1 molar acetic acid and incubated in polylysine, PBS, and/or collagen to coat the nylon. Polystyrene could be similarly treated using sulfuric acid.

In addition, the external surfaces of the three-dimensional framework may be modified to improve the attachment or growth of cells and differentiation of tissue, such as by plasma coating the framework or addition of one or more proteins (e.g., collagens, elastic fibers, reticular fibers), glycoproteins, glycosaminoglycans (e.g., heparin sulfate, chondroitin-4-sulfate, chondroitin-6-sulfate, dermatan sulfate, keratin sulfate), a cellular matrix, and/or other materials such as, but not limited to, gelatin, alginates, agar, agarose, and plant gums, among others.

In some embodiments, the scaffold is comprised of or is treated with materials that render it non-thrombogenic. These treatments and materials may also promote and sustain endothelial growth, migration, and extracellular matrix deposition. Examples of these materials and treatments include but are not limited to natural materials such as basement membrane proteins such as laminin and Type IV collagen, synthetic materials such as ePTFE, and segmented polyurethaneurea silicones, such as PURSPAN (The Polymer Technology Group, Inc., Berkeley, Calif.). These materials can be further treated to render the scaffold non-thrombogenic. Such treatments include antithrombotic agents such as heparin, and treatments which alter the surface charge of the material such as plasma coating.

In some embodiments, the surface of the scaffold is textured. For example, in some aspects of the invention, the scaffold is provided with a groove and ridge pattern. The grooves are preferably less than about 500 microns, more preferably less than about 100 microns, and most preferably between about 10 nanometers and 10 microns. Such "microgrooves" allow the cells to align and/or migrate guided by the surface grooves.

In some embodiments, it is important to re-create in culture the cellular microenvironment found *in vivo,* such that the extent to which the cells of the invention are grown prior to implantation *in vivo* or use *in vitro* may vary. In addition, growth factors, chondrogenic differentiation inducing agents, osteogenic inducing agents, and angiogenic factors may be added to the culture medium prior to, during, or subsequent to inoculation of the cells to trigger differentiation and tissue formation by the MPC or progeny derived therefrom or co-cultures thereof.

The three-dimensional framework may be modified so that the growth of cells and the production of tissue thereon is enhanced, or so that the risk of rejection of the implant is reduced. Thus, one or more biologically active compounds, including, but not limited to, anti-inflammatories, immunosuppressants or growth factors, may be added to the framework.

### Therapeutic Uses for Extracellular Matrix or Cell Lysates

As an alternative to implanting the cells of the invention, or living tissue produced therefrom, a subject in need of tissue repair, replacement, or augmentation may benefit from the administration of a component or product of MEMPs (particularly where they have been genetically modified), such as the extracellular matrix (ECM) or cell lysate produced by those cells.

In some embodiments, after the MEMPs have been cultured *in vitro,* such as, for example, by using a three-dimensional scaffold system described herein, such that a desired amount of ECM has been secreted onto the framework. Once ECM is secreted onto the framework, the cells may be removed. The ECM may be processed for further use, for example, as an injectable preparation.

In some embodiments, the cells are killed and cellular debris (e.g., cellular membranes) is removed from the framework. This process may be carried out in a number of different ways. For example, the living tissue can be flash-frozen in liquid nitrogen without a cryopreservative, or the tissue can be immersed in sterile distilled water so that the cells burst in response to osmotic pressure. Once the cells have been killed, the cellular membranes may be disrupted and cellular debris removed by treatment with a mild detergent rinse, such as EDTA, CHAPS or a zwitterionic detergent. An advantage to using a mild detergent rinse is that it solubilizes membrane-bound proteins, which are often highly antigenic.

Alternatively, the tissue can be enzymatically digested and/or extracted with reagents that break down cellular membranes. Example of such enzymes include, but are not limited to, hyaluronidase, dispase, proteases, and nucleases (for example, deoxyribonuclease and ribonuclease). Examples of detergents include non-ionic detergents such as, for example, alkylaryl polyether alcohol (TRITON™ X-100), octylphenoxy polyethoxy-ethanol (Rohm and Haas Philadelphia, Pa.), BRIJ-35, a polyethoxyethanol lauryl ether (Atlas Chemical Co., San Diego, Calif.), polysorbate 20 (TWEEN 20™), a polyethoxyethanol sorbitan monolaureate (Rohm and Haas), polyethylene lauryl ether (Rohm and Haas); and ionic detergents such as, for example, sodium dodecyl sulphate, sulfated higher aliphatic alcohols, sulfonated alkanes and sulfonated alkylarenes containing 7 to 22 carbon atoms in a branched or unbranched chain.

Scaffolds comprising the ECM may be used therapeutically as described above. Alternatively, ECM may be collected from the scaffold. The collection of the ECM can be accomplished in a variety of ways, depending, for example, on whether the scaffold is biodegradable or non-biodegradable. For example, if the framework is non-biodegradable, the ECM can be removed by subjecting the framework to sonication, high pressure water jets, mechanical scraping, or mild treatment with detergents or enzymes, or any combination of the above.

If the framework is biodegradable, the ECM can be collected, for example, by allowing the framework to degrade or dissolve in solution. Alternatively, if the biodegradable framework is composed of a material that can itself be injected along with the ECM, the framework and the ECM can be processed in toto for subsequent injection. Alternatively, the ECM can be removed from the biodegradable framework by any of the methods described above for collection of ECM from a non-biodegradable framework. All collection processes are preferably designed so as not to denature the ECM or cell lysate produced by the cells of the invention.

Embodiments of the present invention will now be described in detail with reference to the following non-limiting examples.

### MATERIALS AND METHODS

### Subjects, Cell Culture and Antibodies.

BM aspirates were obtained from the posterior iliac crest of normal adult volunteers (20-35 years old) following informed consent, according to procedures approved by the ethics committee of the Royal Adelaide Hospital, South Australia. Bone marrow mononuclear cells (BMMNC) were obtained by centrifugation over Ficoll 1.077 g/ml (Lymphoprep, Nycomed, Oslo, Norway) at 400g for 30 minutes (min) and then washed and resuspended with Hank's buffered saline solution containing 1% bovine serum albumin and 10mM HEPES, pH 7.35 (HBSS). Primary BMSSC cultures were established in □-MEM supplemented with 20% fetal calf serum and 100 □ML-ascorbate-2-phosphate as previously described (Gronthos and Simmons, Blood 85(4):929-940, 1995) for colony efficiency assays, RT-PCR, immunohistochemistry and developmental studies. BMSSC clonal cell lines were generated by limiting dilution from day 14 colonies derived from STRO-1^{bri}/VCAM-1⁺ sorted cells as described below, following subculture in serum replete medium for proliferation, RT-PCR, imunohistochemistry, and developmental studies.

The STRO-1 antibody is available commercially from R&D Systems (Minneapolis, US). Other Antibodies useful in the present invention are set out in Table 1.

### Magnetic-Activated Cell Sorting (MACS).

This was performed as previously described (Gronthos et al., Isolation, Purification and In Vitro Manipulation of Human Bone Marrow Stromal Precursor Cells. In Marrow Stromal Cell Culture. Owen M. and Beresford J.N. (eds). Cambridge University Press UK, Chapter 3, p. 26-42, 1998; Gronthos and Simmons, Blood 85(4): 929-940, 1995). Approximately 1 x 10⁸ BMMNC were incubated with STRO-1 supernatant at a final concentration of 10µg/ml for 60 min on ice. Cells labelled with STRO-1 were washed with HBSS and resuspended in 1 ml of HBSS containing a 1/50 dilution of biotinylated goat anti-mouse IgM (µ-chain specific; Southern Biotechnology Associates, Birmingham, AL) or biotinylated goat anti-mouse IgG (γ-chain specific; Southern Biotechnology Associates, Birmingham, AL) for 45 min on ice, respectively. Following this, the cells were washed twice in MACS buffer (single strength Ca²⁺ and Mn²⁺ free PBS supplemented with 1 % BSA, 5mM EDTA and 0.01 % sodium azide) and resuspended in 900 □µl of MACS buffer to which 100 µl of streptavidin microbeads (Miltenyi Biotec, Bergisch Gladbach, F.R.G.) was added. The cells were further incubated for 15 min on ice after which streptavidin-fluorescein isothiocyanate (FITC) conjugate (1/50; Caltag Laboratories, San Francisco, CA) was added directly to the suspension for an additional 5 min. The cells were separated on a Mini MACS magnetic column (column capacity 10⁷ cells, Miltenyi Biotec) according to the manufacturers recommendations.

### Fluorescence-activated cell sorting (FACS).

The STRO-1⁺ MACS isolated cells were labelled with streptavidin conjugated FITC, then incubated with either purified anti-CD106 (VCAM-1) antibody 6G10 or anti-CD146 (MUC-18) antibody or isotype control 1B5 (10µg/ml) for 30 minutes on ice, washed and incubated with phycoerythrin (PE) conjugated goat anti-mouse IgG antibody (1/50; Southern Biotechnology Associates, Birmingham, AL) for an additional 20 minutes on ice. Cells were sorted using a FACStar^{PLUS} flow cytometer (Becton Dickinson, Sunnyvale, CA). The STRO-1^{bri}/CD106⁺ or STRO-1^{bri}/CD146⁺ cells were cultured in alpha-Modification of Eagle's Medium supplemented with 20% fetal calf serum, L-glutamine 2mM, ascorbate-2-phosphate (100µM) to initiate primary culture in 5% CO₂, at 37°C humidified atmosphere.

### Single and Two-Colour Flow Cytometric Analysis using Indirect Immuofluorescence.

This procedure has been reported previously (Gronthos et al., Isolation, Purification and In Vitro Manipulation of Human Bone Marrow Stromal Precursor Cells. In Marrow Stromal Cell Culture. Owen M. and Beresford J.N. (eds). Cambridge University Press UK, Chapter 3, p. 26-42, 1998). Briefly, primary cultures of MPC or MPC derived cells were liberated by trypsin/EDTA digest then incubated for 30 min on ice. Approximately 2 x 10⁵ cells were washed then resuspended in 200 µl of primary antibody cocktail for 1 hr on ice. The primary antibody cocktail consisted of saturating concentrations of the mouse IgM monoclonal antibody STRO-1 and/or a mouse IgG monoclonal antibody to human alkaline phosphatase (ALP, B4-78). For the staining with antibodies reactive with intracellular antigens the cells were first washed with PBS then permeablized by treatment with 70% ethanol on ice for ten minutes then washed prior to staining. The mouse isotype IgM and IgG negative control Mabs were treated under the same conditions. Following incubation with primary antibodies, cells were washed and exposed to saturating levels of goat anti-mouse IgM µ-chain specific-FITC (1/50 dilution) and either goat anti-mouse IgG γ-specific-PE (1/50 dilution) or anti-rabbit Ig-specific-PE (1/50 dilution) (Southern Biotechnology Associates) in a final volume of 100 µl. The cells were incubated for 45 min on ice, then washed twice then fixed in FAX FIX (PBS supplemented with 1% (v/v), 2% (w/v) D-glucose, 0.01% sodium azide). The cells were then analysed on an Epics®-XL-MCL flow cytometer (Beckman Coulter, Hialeah, FL).

### Carboxyfluorescein Diacetate Succinimidyl Ester (CFSE) Labelling.

The cell-permeant fluorescein-based dye CFSE was used to study division-related phenotypic and functional changes during MPC derived cell development. CFSE covalently attaches to cytoplasmic components of cells, resulting in uniform bright fluorescence, which upon cell division is equally distributed between daughter cells. This technique allows the resolution of up to eight cycles of cell division by flow cytometry. Single cell suspensions of *ex vivo* expanded MPC derived cells were washed once, resuspended in 1 ml of PBS/0.1% BSA and 2 µl of 5 mM CFSE (final 10 µM) was added prior to incubating at 37°C for 10 mins. The staining was quenched by the addition of 5 volumes of ice cold culture medium α-MEM-10 and incubated on ice for 5 mins. The cells were washed three times in the culture medium and then plated at low density 1 X 10⁵ in culture flasks (T-25). At various time points, cells were detached by trypsin-EDTA and analysed by flow cytometric analysis.

### Reverse Transcriptase Polymerase Chain Reaction (RT-PCR) analysis.

Primary MPC derived cultures were liberated by trypsin/EDTA treatment then stained with STRO-1 supernatant as described above. Following washing the cells were incubated with phycoerythrin (PE) conjugated goat anti-mouse IgM antibody (1/50; Southern Biotechnology Associates, Birmingham, AL) for an additional 20 minutes on ice. Cells were sorted using a FACStar^{PLUS} flow cytometer (Becton Dickinson, Sunnyvale, CA). Total cellular RNA was prepared from either 2 x 10⁶ STRO-1^{bri} or STRO-1^{dim} sorted primary cells, chondrocyte pellets and other induced cultures and lysed using RNAzolB extraction method (Biotecx Lab. Inc., Houston, TX), according to the manufacturer's recommendations. RNA isolated from each subpopulation was then used as a template for cDNA synthesis, prepared using a First-strand cDNA synthesis kit (Pharmacia Biotech, Uppsala, Sweden). The expression of various transcripts was assessed by PCR amplification, using a standard protocol as described previously (Gronthos et al., J. Bone and Min. Res. 14:48-57, 1999). Primers sets used in this study are shown in Table 2. Following amplification, each reaction mixture was analysed by 1.5% agarose gel electrophoresis, and visualised by ethidium bromide staining. RNA integrity was assessed by the expression of GAPDH.

### Differentiation of CFU-F in vitro.

We have previously reported the conditions for the induction of human BM stromal cells to develop a mineralized bone matrix *in vitro* cultured in αMEM supplemented with 10% FCS, 100 µM L-ascorbate-2-phosphate, dexamethasone 10⁻⁷ M and 3 mM inorganic phosphate (Gronthos et al., Blood. 84: 4164-4173, 1994). Mineral deposits were identified by positive von Kossa staining. Adipogenesis was induced in the presence of 0.5 mM methylisobutylmethylxanthine, 0.5 µM hydrocortisone, and 60 µM indomethacin as previously described (Gimble, J. M. Marrow stromal adipocytes. In Marrow stromal cell culture. Owen M. and Beresford J.N. (eds). Cambridge: Cambridge University Press UK. Chapter 5, p. 67-87, 1998). Oil Red O staining was used to identify lipid-laden fat cells. Chondrogenic differentiation was assessed in aggregate cultures treated with 10 ng/ml TGF-β3 as described (Pittenger et al., Science, 284:143-147, 1999).

### In vivo assay of bone formation.

The adherent cells derived from STRO-1^{bri}/VCAM-1⁺ cells at passage 2-3 were trypsinised, mixed with 40mg hydroxyapatite/tricalcium phosphate ceramic particles (Zimmer Corporation, Warsaw, IN) and then implanted into subcutaneous pockets on the dorsal surface of two month old SCID mice as described previously (Gronthos et al., Proceedings of the National Academy of Sciences (USA), 97 (25): 13625-13630, 2000). These procedures were performed in accordance to specifications of an approved animal protocol (Adelaide University AEC# M/079/94). Implants were recovered after 6-8 weeks, fixed in 4% paraformaldehyde for 2 days, then decalcified for a further ten days in 10% EDTA prior to embedding in paraffin. For histological analysis, 5 µm sections of the implants were prepared and stained with haematoxylin and eosin (Gronthos et al., Proceedings of the National Academy of Sciences (USA), 97 (25): 13625-13630, 2000).

*Neural Tissue Development.* Monolayer cultures are grown in Neuroblast A medium (Invitrogen/GIBCO) + 5% horse serum, 1% fetal calf serum, L-glutamine (2mM), transferrin (100µg/ml), insulin (2µg/ml), retinoic acid 0.5 mM, brain-derived neurothrophic factor (10ng/ml).

*Fat Development.* Monolayer cultures are grown in alpha-Modification of Eagle's Medium (JRH) supplemented with 10% fetal calf serum, L-glutamine 2mM, ascorbate-2-phosphate (100µM), 0.5 mM methylisobutylxanthine, 0.5 mM hydrocortisone, 60 mM indomethicin.

*Cartilage Development:* Pellet cultures in polypropylene tubes are grown in alpha-Modification of Eagle's Medium supplemented with 1% bovine serum albumin, transferrin (100µg/ml), insulin (2µg/ml), L-glutamine (2mM), ascorbate-2-phosphate (100 µM/ml), dexamethasone (10⁻⁸M), with BMP-7(50ng/ml), TGFβ₃ (10ng/ml).

*Skeletal*/*Cardiac Muscle Development.* Monolayer cultures are grown in alpha-Modification of Eagle's Medium supplemented with 10% fetal calf serum,L-glutamine (2mM), ascorbate-2-phosphate (100µM/ml), and 5-azacytodine (5µM/ml).

*Epithelial Development.* Monolayer cultures are grown in keratinocyte basal medium (Clontenics) supplemented with Bovine Pituitary Extract (50µg/ml), epidermal growth factor (10ng/ml), Hydrocortisone (0.5µg/ml), Insulin (5µg/ml).

*Osteoblasts, Tendon, Ligament or Odontoblast Development.* Monolayer cultures are grown in alpha-Modification of Eagle's Medium supplemented with 10% fetal calf serum, L-glutamine 2mM, ascorbate-2-phosphate (100µM), Dexamethasone (10⁻⁷M) and BMP-2 (50ng/ml)

*Pericyte or Smooth Muscle Cell Development.* Cultures of 20,000 *ex vivo* cultured MPCs per well are grown in alpha-Modification of Eagle's Medium supplemented with 10% fetal calf serum, L-glutamine 2mM, ascorbate-2-phosphate (100µM), platelet derived growth factor-BB (10ng/ml) suspended over 200□1 of matrigel in 48-well plates.

### EXAMPLE 1: Stro-1^{dim} cultured cells are more committed while Stro-1^{bri} cells are less committed precursor cells.

We have previously reported that multipotential mesenchymal precursor cells (MPC) can be purified from adult human bone marrow mononuclear cells based on the phenotype STRO-1^{bri}/VCAM-1 (CD106)⁺ or STRO-1^{bri}/MUC-18 (CD146)⁺ (Gronthos et al. J. Cell Sci 116:1827-1835, 2003; Shi and Gronthos JBMR 18(4): 696-704, 2003; PCTAU2004/000416). The MPC population can be readily expanded *in vitro* under defined culture conditions (Gronthos et al. J. Cell Sci 116:1827-1835, 2003). We now present data characterising the *ex vivo* expanded MPC progeny based on markers associated with different cell lineages, at both the mRNA and protein level, using reverse transcription-polymerase chain reaction (RT-PCR) and flow cytometric analysis, respectively. Whilst, all freshly isolated bone marrow MPC express STRO-1 at high levels (Stro-1^{bri}), the majority of cells down regulate STRO-1 expression (Stro-1^{dim}) following *ex vivo* expansion (Gronthos et al. J. Cell Sci 116:1827-1835, 2003). In the first series of experiments, semi-quantitative RT-PCR analysis was employed to examine the gene expression profile of various lineage-associated genes expressed by STRO-1^{dim} or STRO-1^{bri} populations, isolated by fluorescence activated cell sorting (Figure 1A). Relative gene expression for each cell marker was assessed with reference to the expression of the house-keeping gene, GAPDH, using ImageQant software (Figure 1B, C). In addition, dual-colour flow cytometric analysis was used to examine the protein expression profile of *ex vivo* expanded MPC based on their expression of a wider range of cell lineage-associated markers in combination with the STRO-1 antibody (Figure 2). A summary of the general phenotype based on the gene and protein expression of STRO-1^{dim} and STRO-1^{bri} cultured cells is presented in Table 3. The data indicate that *ex vivo* expanded STRO-1^{bri} MPC exhibit differentially higher expression of markers associated with perivascular cells, including angiopoietin-1, VCAM-1, SDF-1, IL-1β, TNFα, and RANKL. Conversely, STRO-1^{dim} *ex vivo* expanded cells expressed higher levels of nestin, GFAP, osterix, osteocalcin, SOX9, GATA-4, leptin, and smooth muscle myosin heavy chain. It therefore appears that *ex vivo* expanded STRO-1^{bri} MPC exhibit a more immature and perivascular-like phenotype in comparison to STRO-1^{dim} cells which exhibit a phenotype characteristic of more committed precursor cell types including chondroblasts, osteoblasts, adipoblasts, epithelial cells, neural progenitors and cardiomyoblasts. Comparisons between the protein and gene expression profiles of STRO-1^{dim} and STRO-1^{bri} cultured cells are summarised in Tables 3, 4 and 5. A comparison of maker expression between freshly isolated MPCs and STRO-1^{bri} cultured progeny of MPCs (MEMPs) is shown in Table 6.

### EXAMPLE 2: Differential capacity of STRO-1^{dim} and STRO-1^{bri} cultured cells (MEMPs) to differentiate in vitro.

We next examined whether the observed differences in the gene and protein expression profiles of STRO-1^{dim} and STRO-1^{bri} cultured cells was reflective of any functional differences in their capacity to differentiate into multiple cell lineages. Cultures of *ex vivo* expanded STRO-1^{bri}/CD146⁺ derived cells were isolated by FACS based on their expression of STRO-1 antigen as described above. FACS isolated STRO-1^{dim} and STRO-1^{bri} cultured cells were subsequently plated under inductive conditions for fat (Figure 3), bone (Figure 4) and cartilage (Figure 5) formation. In all cases STRO-1^{bri} cultured cells showed a higher capacity to form fat, bone and cartilage under the specified conditions when compared with STRO-1^{dim} cultured cells. The data from these experiments, substantiate the gene and protein expression results obtained above, demonstrating that STRO-1^{bri} cultured cells are a primitive population containing a high proportion of less committed precursor cells that can be influenced to differentiate towards any specified cell lineage under the appropriate culture conditions (Figures 3, 4, 5) and may be referred to as MPC. Conversely, the STRO-1^{dim} cultured cells contain a high proportion of committed cells representative of various lineages and may be referred to as TSCC. It is proposed that the Stro-1^{dim} population is heterogenous comprising cells separately committed to range of different tissue types.

### EXAMPLE 3: STRO-1^{bri} cells (MEMPs) can modify the growth potential of Tissue Specific Committed Cells (TSCC) in vitro and in vivo.

The identification of the two different *ex vivo* expanded MPC derived cell populations representative of different developmental stages has significant implications in the use of whole cultured preparations derived from Stro-1^{bri} cells for clinical therapies. Initial studies were design to examine the influence of primitive, less committed STRO-1^{bri} cultured MPC on the growth of more mature and committed STRO-1^{dim} cultured TSCC. Experiments were designed to add increasing percentages of FACS isolated STRO-1^{bri} cultured MPC with FACS isolated STRO-1^{dim} cultured TSCC, previously labelled with a fluorescent tag, CFSE. Figure 6 shows that the proliferation of labelled STRO-1^{dim} cells is effected by the presence of unlabelled STRO-1^{bri} cells. When a CFSE labelled cell divides the two daughter cells contain half the fluorescence of the parental cell. Therefore, different generations of daughter cells are represented as fluorescent distributions with proportionate ever decreasing fluorescence intensity, where the curve on the far right of the histogram (intersected by vertical line) represents the point of the initial STRO-1^{dim} population (Figure 6). The data demonstrated that a higher proportion of STRO-1^{dim} cells were stimulated to increase their proliferation rates, where more cells were shown to be undergoing at least 3 to 4 divisions, following the addition of greater than 5% STRO-1^{bri} cells. Therefore, it follows that in order to get a sustainable and efficient *ex vivo* expansion of unfractionated MPC derived cells the cultures require the presence of greater than 5% of STRO-1^{bri} cells within the population.

Further investigations were performed to determine whether more primitive, less committed STRO-1^{bri} cultured MPC could also influence the proliferation capacity of TSCC *in vivo.* Two *in vivo* models were used to address this question. The first model employed athymic nude rats that had undergone ligation of the left anterior descending (LAD) coronary artery and injected 48 hours later with saline, FACS isolated cultured human STRO-1^{dim} and STRO-1^{bri} cells and fresh aspirates of STRO-1 depleted bone marrow mononuclear cells (Figure 7). After two weeks animals were sacrificed, and cardiac tissues were fixed and concomitantly stained with two monoclonal antibodies: the first being selectively reactive with the rat, but not the human, Ki67 antigen, and the second being reactive with the cardiomyocyte marker troponin I. Dually stained cells, indicative of proliferating rat cardiomyocytes, were detected by immunoperoxidase technique. Animals receiving STRO-1^{bri} human cells demonstrated 2.5-5 fold higher numbers of proliferating rat cardiomyocytes compared with control animals receiving saline or STRO-1^{dim} human cells (Figure 7).

The second model utilized athymic nude rats injected subcutaneously with rat glioblastoma tumor cells, which constitutively secrete VEGF. Two weeks later, the rats received intra-tumor injections with either saline, FACS isolated human STRO-1^{dim} or STRO-1^{bri} human cells (Figure 8). One week later, animals were sacrificed, and tumor tissues were fixed and concomitantly stained with two monoclonal antibodies: the first being reactive with the alpha-smooth muscle actin antigen expressed by smooth muscle cells, and the second being reactive with the vWF antigen expressed by vascular endothelial cells. Dually stained structures, indicative of arterioles and arteries containing both endothelium and smooth muscle, were detected by immunoperoxidase technique. Animals receiving STRO-1^{bri} human cells demonstrated 3.5-8 fold higher numbers of arterioles and arteries at the site of cellular injection in the tumors compared with control animals receiving saline or STRO-1^{dim} human cells (Figure 8). No differences were seen at sites distal to where the human cells had been injected.

### EXAMPLE 4: Increase in the number of STRO-1^{bri} MEMPs in cell cultures derived from STRO-1 positive cells.

After demonstrating the capacity of STRO-1^{bri} cultured MEMPs to increase the proliferation of more TSCC we next examined the effect of a range of growth factors to increase the proportion of *ex vivo* expanded STRO-1^{bri} MPC (Figure 9). Established cultures derived from STRO-1^{bri}/CD146⁺ isolated bone marrow cells were grown in basal medium supplemented with 10% FCS (A) or a range of factors, including 1x10⁻⁸ M 1α,25-dihydroxyvitamin D₃ (1,25D) (B) 10ng/ml Platelet derived growth factor (PDGF) (C), 10 ng/ml Tumor necrosis factor-alpha (TNF-α) (D); 10 ng/ml interleukin-1β (IL-1β) (E) and 30 ng/ml stromal derived factor 1-alpha (SDF-1α) (F), for 5 days, stained with STRO-1 mAb. (Figure 9). These factors were found to greatly enhance the number of number of STRO-1^{bri} MPC *in vitro.*

To investigate the mechanisms of how these factors enhanced the percentage of STRO-1^{bri} expressing cells following *ex vivo* expansion, cultured Stro-1^{bri} were labelled with CFSE as described in the method then exposed to the various factors. Figure 10 shows a representative experiment, where IL-1β increased the proliferative potential of MPC labelled with CFSE as described in the methods. Cells were cultured in the presence of 10 ng/ml IL-1β for 5 days, stained with STRO-1 mAb and analysed as described above. IL-1β was found to enhance the number of MPC divisions by increasing the number of bright STRO-1⁺ osteoprogenitor cells. Similar results were also obtained 1,25D, PDGF-BB, TNF-α, IL-1β, and SDF-1α were used to stimulate MPCs.

### EXAMPLE 5: Increasing proliferation of Stro-1^{bri} cells also increases the number of Stro-1^{dim} cells.

The ability to enhance the proportion of STRO-1^{bri} cultured MEMPs in the presence of various factors also correlated with an increase in the number of Stro-1^{dim} cells. For example STRO-1^{bri}/Alk Phos⁺ cells (Figure 10B) a phenotype consistent with pre-osteoblastic cells (Gronthos et al., J Bone Miner Res. 14: 47-56, 1999; Pan et al., Bone 34(1):112-23, 2004). We therefore examined whether this change in phenotype also correlated with an increased capacity of the induced STRO-1^{bri} MPC to differentiate into bone forming cells, osteoblasts. Figure 11 shows that IL-1β not only stimulated STRO-1 positive MPC proliferation, but also enhanced their bone forming potential in the presence of the osteoinductive agent, dexamethasone. IL-1β at concentration 0.01ng/ml significantly increased MPC number to 136.6±1.2% of untreated control cultures (Figure 11A). A plateau effect was achieved at concentrations greater than 0.1 ng/ml. *Ex vivo* expanded progeny of MPC were seeded into 24-well plates in the presence of osteoinductive conditions, as described in the methods. The cells were also treated with IL-1β at a concentration 10 ng/ml and cultures were "fed" weekly with fresh medium containing IL-1β. The absolute extracellular matrix calcium concentration was determined according to the methods. The results showed that mineral deposition was increased in cells treated with IL-1β (Figure 11C) compared to untreated cells (Figure 11B). The calcium level in IL-1β treated cells was significantly higher than that in untreated cells at both week 4 and week 6.

Data presented in Figure 12 suggests that IL-1β stimulated the proliferation and STRO-1^{Bri} MPC, resulting in an expansion of oetoprogenitors, whilst later addition of a secondary differentiation agent, dexamethasone, induced alkaline phosphatase (ALP) expression and loss of STRO-1 expression effectively enhancing the number of functional osteoblasts *in vitro.* The concept that, different factors can expand and regulate the STRO-1^{Bri} MPC population was further tested *in vivo.* Semi-confluent secondary cultures of *ex vivo* expanded from Stro-1^{bri} MPC, were cultured in the presence or absence of PDGF-BB (10ng/ml) an additional factor known to enhance the number of *ex vivo* expanded STRO-1^{bri} MPC (please refer to Figure 9C). PDGF-induced and non-induced cell preparations were subsequently co-transplanted with hydroxyapetite/tricalcium phosphate particles (HA/TCP) into immunocompromised mice as described in the methods. After eight weeks, examination of the harvested transplants showed that cultures pre-treated with PDGF-BB exhibited significantly more ectopic bone formation (Figure 13C) when compared with untreated control cultures (Figure 13B) as quantitated by Scion Imaging (Figure 13A).

### EXAMPLE 6: Uncommitted STRO-1^{bri} MPC which lack detectable expression of ALP persist in ex vivo cultures of STRO-1-selected BM-derived MPC.

Aspirates of human BM were prepared as described in the methods and the MPC recovered by MACS selection using the mAb STRO-1. Using indirect immunofluorescence and flow cytometry, the MACS positive fraction (cells used to establish the initiating or P0 culture) was assessed for the proportion of cells which expressed the STRO-1 antigen at high levels (STRO-1^{Bright}) and was found to be 22.4% of the total population (data not shown). Theses cells were then plated at 1 x 10⁴ cells per cm² and cultured in serum replete medium until they achieved a confluence of 80-90%, as previously described (Gronthos et al. Journal of Cell Science 116: 1827-1835, 2003). At each passage, cells were detached as described in the Methods and reseeded at the 1 x 10⁴ cells per cm². Cell samples form each passage were stained for their expression of STRO-1 and the TSCC marker, alkaline phosphatase (ALP). As shown in Figure 14, after 4 passages, whilst the proportion of cells expressing STRO-1 at high levels (and lacking appreciable levels of the TSSC marker, ALP) had dropped to 12.7%, these cultures still contained considerable numbers of STRO-1^{bri} ALP⁻ MEMPs.

**Table 1. Antibodies used in this patent**

| **CELL TYPE** | **ANTIGEN** | **SOURCE ISOTYPE DILUTION** |
|---|---|---|
| Skeletal Muscle | Myo D | Santa Cruz Rabbit Ig 1/50 |
| | Desmin | DAKO IgG1 10ug/ml |
| Smooth Muscle | SMMHC | Sigma mIgG1 Acites 1/500 |
| | SMHC -FAST | Sigma mIgG1 10ug/ml |
| | alphaSMAC | DAKO mIgG2a 10ug/ml |
| | PDGF-R | Pharmigen mIgG 10ug/ml |
| | Vimentin | DAKO mIgG1 10ug/ml |
| Chondrocytes | Type II Collagen | Chemicon mIgG1 10 ug/ml |
| | Collagen IX | Chemicon mIgG2A 10 ug/ml |
| | Aggrecan | Chemicon mIgG1 10ug/ml |
| | Link Protein | DSHB mouse IgG2b 10ug/ml |
| | S-100 | Chemicon rabbit Ig 1/100 |
| | Biglycan | Dr. Larry Fisher NIH RABBIT Ig 1/500 |
| Basal Fibroblasts | Laminin | Chemicon mIgG1 10ug/ml |
| | Type IV Collagen | DAKO mIgG1 10ug/ml |
| | Versican | DHSB 12C5 IgG1 10ug/ml |
| Endothelial Cells | vWF | DAKO IgG1 mouse |
| | VCAM-1 | Chemicon IgG1 6G10 10ug/ml |
| | Endoglin | BD IgG1 10ug/ml |
| | MUC18 | In house CC9 IgG2a 10ug/ml |
| | CD31 | DAKO IgG 10ug/ml |
| | CD34 | DAKO mIgG1 10ug/ml |
| | SDF-1 | R&D IgG1 10ug/ml |
| Cardiomyocytes | calponin | Chemicon IgG1 10ug/ml |
| | Troponin I | Accurate Chem and Sci Corp IgG1 10ug/ml |
| | Troponin C | Chemicon mIgG2a 10ug/ml |
| Neurons | NCAM | DAKO IgG2a 10ug/ml |
| | GFAP | DAKO mIgG1 10ug/ml |
| | Neuroanalase | DAKO RABBIT Ig 1/200 |
| | Neurofilament | DAKO IgG1 10ug/ml |
| Bone | AP | DSHB mIgG1 10ug/ml |
| | Type I Collagen | CHEMICON mouse IgG 10ug/ml |
| | CBFA 1 | Alpha Diagnostic RABBIT Ig 1/200 |
| | OCN | Chemicon RABBIT Ig 1/200 |
| | OPG | R&D IgG2b 10ug/ml |
| | RANKL | R&D IgG2a 10ug/ml |
| | Annexin II | Santa Cruz RABBIT Ig 1/100 |
| Fat | CEPBalpha | Santa Cruz RABBIT Ig 1/200 |
| | PPARgamma | Santa Cruz RABBIT Ig 1/200 |
| | Leptin | Chemicon IgG 10ug/ml |
| Epithelial Cells | Keratin 14 | DAKO mIgG 10ug/ml |
| | Cytokeratin 10+13 | DAKO mIgG2a 10ug/ml |
| | EGFR | Pharmingen mIgG 10ug/ml |
| Fibroblast | Collagen III | Chemicon mIgG1 10ug/ml |
| | NGFR | Santa Cruz mIgG1 10ug/ml |
| | Fibroblast marker | SIGMA mIgG 10ug/ml |
| Haematopoietic | CD14 | DAKO IgG2a 10ug/ml |
| | CD45 | DAKO IgG1 10ug/ml |
| | Glycophorin-A | DAKO IgG 10ug/ml |

**Table 2. RT-PCR primers and conditions for the specific amplification of human mRNA**

| **Target Gene** | **Sense/ Antisense (5'-3') Primer Sequences** | **Product Size** |
|---|---|---|
| **GAPDH** | CACTGACACGTTGGCAGTGG (SEQ ID NO:1)/ CATGGAGAAGGCTGGGGCTC (SEQ ID NO:2) | 417 |
| **SDF-1** | GAGACCCGCGCTCGTCCGCC (SEQ ID NO:3)/ GCTGGACTCCTACTGTAAGGG (SEQ ID NO:4) | 364 |
| **IL-1β** | AGGAAGATGCTGGTTCCCTCTC (SEQ ID NO:5)/ CAGTTCAGTGATCGTACAGGTGC (SEQ ID NO:6) | 151 |
| **FLT-1** | TCACTATGGAAGATCTGATTTCTTACAGT (SEQ ID NO:7)/ GGTATAAATACACATGTGCTTCTAG (SEQ ID NO:8) | 380 |
| **TNF-α** | TCAGATCATCTTCTCGAACC (SEQ ID NO:9)/ CAGATAGATGGGCTCATACC (SEQ ID NO:10) | 361 |
| **KDR** | TATAGATGGTGTAACCCGGA (SEQ ID NO:11)/ TTTGTCACTGAGACAGCTTGG (SEQ ID NO:12) | 450 |
| **RANKL** | AACAGGCCTTTCAAGGAGCTG (SEQ ID NO:13)/ TAAGGAGGGGTTGGAGACCTCG (SEQ ID NO:14) | 538 |
| **Leptin** | ATGCATTGGGAACCCTGTGC (SEQ ID NO:15)/ GCACCCAGGGCTGAGGTCCA (SEQ ID NO:16) | 492 |
| **CBFA-1** | GTGGACGAGGCAAGAGTTTCA (SEQ ID NO:17)/ TGGCAGGTAGGTGTGGTAGTG (SEQ ID NO:18) | 632 |
| **PPARγ2** | AACTGCGGGGAAACTTGGGAGATTCTCC (SEQ ID NO:19)/ AATAATAAGGTGGAGATGCAGGCTCC (SEQ ID NO:20) | 341 |
| **OCN** | ATGAGAGCCCTCACACTCCTC (SEQ ID NO:21)/ CGTAGAAGCGCCGATAGGC (SEQ ID NO:22) | 289 |
| **MyoD** | AAGCGCCATCTCTTGAGGTA (SEQ ID NO:23)/ GCGAGAAACGTGAACCTAGC (SEQ ID NO:24) | 270 |
| **SMMHC** | CTGGGCAACGTAGTAAAACC (SEQ ID NO:25)/ TATAGCTCATTGCAGCCTCG (SEQ ID NO:26) | 150 |
| **GFAP** | CTGTTGCCAGAGATGGAGGTT (SEQ ID NO:27)/ TCATCGCTCAGGAGGTCCTT (SEQ ID NO:28) | 370 |
| **Nestin** | GGCAGCGTTGGAACAGAGGTTGGA (SEQ ID NO:29)/ CTCTAAACTGGAGTGGTCAGGGCT (SEQ ID NO:30) | 460 |
| **SOX9** | CTCTGCCTGTTTGGACTTTGT (SEQ ID NO:31)/ CCTTTGCTTGCCTTTTACCTC (SEQ ID NO:32) | 598 |
| **Collagen type X** | AGCCAGGGTTGCCAGGACCA (SEQ ID NO:33)/ TTTTCCCACTCCAGGAGGGC (SEQ ID NO:34) | 387 |
| **Aggrecan** | CACTGTTACCGCCACTTCCC (SEQ ID NO:35)/ ACCAGCGGAAGTCCCCTTCG (SEQ ID NO:36) | 184 |

**Table 3. Summary of the Relative Gene Expression in STRO-1^{Bri} and STRO-1^{Dim} populations. A list of genes which displayed measurable and differential expression between the STRO-1^{Bri} and STRO-1^{Dim} populations as determined by reverse transcription-PCR are presented . Values represent the relative gene expression with reference to the house-keeping gene, GAPDH.**

| | | **Gene Expression relative to GAPDH** | |
|---|---|---|---|
| **Tissue** | **Marker** | **STRO-1^{Bri}** | **STRO-1^{Dim}** |
| **Neurons** | *GFAP (Glial Fibrillary Acidic Protein)* | 0.1 | 0.7 |
| **Bone** | *OCN (Osteocalcin)* | 1.1 | 2.5 |
| | *OSX (Osterix)* | 0.4 | 1.3 |
| | *CBFA-1 (Core Factor Binding Protein-1)* | 0.3 | 0.6 |
| | *RANKL (Receptor Activator of Nuclear Factor κ B)* | 1.6 | 0.3 |
| **Fat** | *Leptin* | 3.1 | 4.2 |
| **Cardiomyocytes** | *GATA-4* | 1.1 | 2.9 |
| **Endothelial cells** | *Ang-1 (Angiopoietin-1)* | 1.5 | 0.8 |
| | *SDF-1-alpha (Stromal Derived factor-1-alpha)* | 3.2 | 0.1 |
| **Chondrocytes** | *Sox 9* | 0.3 | 1.1 |
| | *COL X (Collagen X)* | 3.5 | 2.8 |
| **Pro-inflammatory Cytokines** | *TNF-alpha (Tumour necrosis alpha)* | 1.7 | 0.9 |

**Table 4. Summary of the Relative Protein Expression in STRO-1^{bri} and STRO-1^{dim} populations. A list of proteins which displayed differential expression between the STRO-1^{bri} and STRO-1^{dim} populations as determined by flow cytometry are presented. Values represent the relative mean fluorescence intensity of staining as described in Figure 2.**

| | | **Mean Fluorescence Intensity** | |
|---|---|---|---|
| **Tissue** | **Marker** | **STRO-1^{bri}** | **STRO-1^{dim}** |
| **Neurons** | *Neurofilament* | 1.7 | 20.5 |
| **Bone** | *ALK PHOS (Alkaline Phophatase)* | 5.7 | 44.5 |
| | *RANKL (Receptor Activator of Nuclear Factor κ B)* | 658.5 | 31.0 |
| **Epithelial Cells** | ***CytoKeratin** 10*+*13* | 1.2 | 23.3 |
| | *Cytokeratin 14* | 1.8 | 8.8 |
| **Smooth Muscle** | *α-SMA (Alpha Smooth Muscle Actin*) | 318.0 | 286.0 |
| **Chondrocytes** | *Byglycan* | 84.4 | 65.9 |
| **Basal Fibroblast** | *Tenascin C* | 22.2 | 6.9 |
| **Cardiomyocyte** | *Troponin C* | 2.5 | 15.0 |

**Table 5: Comparison of marker expression between MEMPs (STRO-1^{bri}) and Tissue Specific Committed Cells (TSCCs) (STRO-1^{dim})**

| **Marker** | **TSCC** | **MEMP** |
|---|---|---|
| Stro-1 | + | +++ |
| N eurofilament | ++ | + |
| OCN | ++ | + |
| OCX | ++ | + |
| CBFA-1 | ++ | + |
| RANKL | + | ++ |
| Leptin | ++ | + |
| GATA-4 | ++ | + |
| SDF-1 | + | ++ |
| Tenascin-C | + | ++ |
| α-SMA | + | ++ |
| Sox9 | ++ | + |

**Table 6: Comparison of marker expression between freshly isolated MPCs and MEMPs**

| **Marker** | **Freshly isolated MPC** | **MEMP** |
|---|---|---|
| Stro-1 | +++ | +++ |
| Ki67 | - | ++ |
| TERT activity | +++ | - |
| CD49a | - | - |
| Alk Phos | + | - |
| CD44 | - | ++ |
| CD18 | + | - |
| CD49c/CD29, VLA-3, α3β1 | - | + |

## Claims

1. An *in vitro* method of increasing the generation of multipotential expanded mesenchymal precursor cell progeny (MEMPs) that have the phenotype Stro-1^{bri}, ALP⁻, the method comprising culturing STRO-1^{bright} ALP⁺ mesenchymal progenitor cells (MPC) in the presence of one or more stimulatory factors selected from the group consisting of 1α,25-dihydroxyvitamin D₃ (1,25D), tumor necrosis factor α (TNF-α), and interleukin-1β (IL-1β).

2. The method of claim 1 wherein the STRO-1^{bright} ALP⁺ MPC thereof are cultured in the presence of two or more stimulatory factors.

3. The method of any one of claim 1 or claim 2 wherein the STRO-1^{bright} ALP⁺ MPC have been expanded *ex vivo.*

4. The method of any one of claims 1 to 3 wherein the STRO-1^{bright} ALP⁺ MPC are an unexpanded population of isolated MPC.

5. The method of any one of claims 1 to 4 wherein the stimulation results in an increase in MPC progeny that have the phenotype Stro-1^{bri}, ALP⁻ of more than 10% relative to non stimulated controls.

6. The method of claims 1 to 5 wherein the stimulation results in an increase in MPC progeny that have the phenotype Stro-1^{bri}, ALP⁻ of more than 50% relative to non stimulated controls.

7. The method of any one of claims 1 to 6 wherein the STRO-1^{bright} ALP⁺ MPC are derived from any one or more tissues consisting of the group comprising bone marrow, dental pulp cells, adipose tissue and skin, adipose tissue, teeth, dental pulp, skin, liver, kidney, heart, retina, brain, hair follicles, intestine, lung, spleen, lymph node, thymus, pancreas, bone, ligament, bone marrow, tendon and skeletal muscle.

## Patentansprüche

1. *In vitro*-Verfahren zur Steigerung der Erzeugung multipotenter expandierter mesenchymaler Vorläuferzellenabkömmlinge (MEMPs) mit dem Phänotyp Stro-1^{bri}, ALP⁻, wobei das Verfahren das Kultivieren von STRO-1^{bright} ALP⁺ mesenchymalen Vorläuferzellen (MPCs) in Gegenwart eines oder mehrerer Stimulationsfaktoren umfasst, die ausgewählt sind aus der Gruppe bestehend aus 1α,25-Dihydroxyvitamin D₃ (1,25D), Tumornekrosefaktor α (TNF-α) und Interleukin-1β (IL-1β).

2. Verfahren nach Anspruch 1, wobei die STRO-1^{bright} ALP⁺ MPCs daraus in Gegenwart von zwei oder mehr Stimulationsfaktoren kultiviert werden.

3. Verfahren nach Anspruch 1 oder 2, wobei die STRO-1^{bright} ALP⁺ MPCs *ex vivo* expandiert worden sind.

4. Verfahren nach einem der Ansprüche 1 bis 3, wobei die STRO-1^{bright} ALP⁺ MPCs eine nicht expandierte Population isolierter MPCs sind.

5. Verfahren nach einem der Ansprüche 1 bis 4, wobei die Stimulation zu einer Steigerung der MPC-Abkömmlinge führt, welche den Phänotyp Stro-1^{bri}, ALP⁻ von nicht mehr als 10% im Verhältnis zu nicht stimulierten Kontrollen aufweisen.

6. Verfahren nach einem der Ansprüche 1 bis 5, wobei die Stimulation zu einer Steigerung der MPC-Abkömmlinge führt, welche den Phänotyp Stro-1^{bri}, ALP⁻ von nicht mehr als 50% im Verhältnis zu nicht stimulierten Kontrollen aufweisen.

7. Verfahren nach einem der Ansprüche 1 bis 6, wobei die STRO-1^{bright} ALP⁺ MPCs von einem oder mehreren beliebigen Geweben aus der Gruppe stammen, die folgendes umfasst: Knochenmark, dentale Pulpazellen, Fettgewebe und Haut, Fettgewebe, Zähne, dentale Pulpa, Haut, Leber, Niere, Herz, Netzhaut, Gehirn, Haarfollikel, Darm, Lunge, Milz, Lymphknoten, Thymus, Pankreas, Knochen, Ligament, Knochenmark, Sehne und Skelett-Muskulatur.

## Revendications

1. Procédé *in vitro* d'augmentation de la génération de descendance cellulaire de précurseurs mésenchymateux développés multipotents (MEMP) qui ont le phénotype Stro-1^{bri}, ALP⁻, le procédé comprenant la culture de cellules progénitrices mésenchymateuses (MPC) STRO-1^{bright} ALP⁺ en présence d'un ou de plusieurs facteurs stimulants choisis dans le groupe constituté par 1α,25-dihydroxyvitamine D₃ (1,25D), facteur de nécrose tumorale α (TNF-α), et interleukine-1β (IL-1β).

2. Procédé selon la revendication 1, les MPC STRO-1^{bright} ALP⁺ étant cultivées en présence d'au moins deux facteurs stimulants.

3. Procédé selon la revendication 1 ou revendication 2, les MPC STRO-1^{bright} ALP⁺ ayant été développées *ex vivo.*

4. Procédé selon l'une quelconque des revendications 1 à 3, les MPC STRO-1^{bright} ALP⁺ étant une population non-développée de MPC isolées.

5. Procédé selon l'une quelconque des revendications 1 à 4, la stimulation entraînant une augmentation de la progéniture MPC ayant le phénotype Stro-1^{bri}, ALP⁻ de plus de 10 % par rapport aux témoins non stimulés.

6. Procédé selon les revendications 1 à 5, la stimulation entraînant une augmentation de la progéniture MPC ayant le phénotype Stro-1^{bri}, ALP⁻ de plus de 50 % par rapport aux témoins non stimulés.

7. Procédé selon l'une quelconque des revendications 1 à 6, les MPC STRO-1^{bright} ALP⁺ étant dérivées d'un ou plusieurs tissus parmi le groupe constitué de moelle osseuse, cellules de pulpe dentaire, peau et tissu adipeux, tissu adipeux, dents, pulpe dentaire, peau, foie, rein, coeur, rétine, cerveau, follicules pileux, intestin, poumon, rate, ganglion lymphatique, thymus, pancréas, os, ligament, moelle osseuse, tendon et muscle squelettique.


### SEQUENCE LISTING

<110> Angioblast Systems, Inc.
<120> Multipotential Expanded Mesenchymal Precursor Cell Progeny (MEMP) and uses thereof
<130> 503851
<160> 36
<170> PatentIn version 3.3
<210> 1
   <211> 20
   <212> DNA
   <213> Artificial
<220>
   <223> GAPDH sense oligonucleotide
<400> 1
   cactgacacg ttggcagtgg 20
<210> 2
   <211> 20
   <212> DNA
   <213> Artificial
<220>
   <223> GAPDH antisense oligonucleotide
<400> 2
   catggagaag gctggggctc 20
<210> 3
   <211> 20
   <212> DNA
   <213> Artificial
<220>
   <223> SDF-1 sense oligonucleotide
<400> 3
   gagacccgcg ctcgtccgcc 20
<210> 4
   <211> 21
   <212> DNA
   <213> Artificial
<220>
   <223> SDF-1 antisense oligonucleotide
<400> 4
   gctggactcc tactgtaagg g 21
<210> 5
   <211> 22
   <212> DNA
   <213> Artificial
<220>
   <223> IL-1beta sense oligonucleotide
<400> 5
   aggaagatgc tggttccctc tc 22
<210> 6
   <211> 23
   <212> DNA
   <213> Artificial
<220>
   <223> IL-1beta antisense oligonucleotide
<400> 6
   cagttcagtg atcgtacagg tgc 23
<210> 7
   <211> 29
   <212> DNA
   <213> Artificial
<220>
   <223> FLT-1 sense oligonucleotide
<400> 7
   tcactatgga agatctgatt tcttacagt 29
<210> 8
   <211> 25
   <212> DNA
   <213> Artificial
<220>
   <223> FLT-1 antisense oligonucleotide
<400> 8
   ggtataaata cacatgtgct tctag 25
<210> 9
   <211> 20
   <212> DNA
   <213> Artificial
<220>
   <223> TNF-alpha sense oligonucleotide
<400> 9
   tcagatcatc ttctcgaacc 20
<210> 10
   <211> 20
   <212> DNA
   <213> Artificial
<220>
   <223> TNF-alpha antisense oligonucleotide
<400> 10
   cagatagatg ggctcatacc 20
<210> 11
   <211> 20
   <212> DNA
   <213> Artificial
<220>
   <223> KDR sense oligonucleotide
<400> 11
   tatagatggt gtaacccgga 20
<210> 12
   <211> 21
   <212> DNA
   <213> Artificial
<220>
   <223> KDR antisense oligonucleotide
<400> 12
   tttgtcactg agacagcttg g 21
<210> 13
   <211> 21
   <212> DNA
   <213> Artificial
<220>
   <223> RANKL sense oligonucleotide
<400> 13
   aacaggcctt tcaaggagct g 21
<210> 14
   <211> 22
   <212> DNA
   <213> Artificial
<220>
   <223> RANKL antisense oligonucleotide
<400> 14
   taaggagggg ttggagacct cg 22
<210> 15
   <211> 20
   <212> DNA
   <213> Artificial
<220>
   <223> Leptin sense oligonucleotide
<400> 15
   atgcattggg aaccctgtgc 20
<210> 16
   <211> 20
   <212> DNA
   <213> Artificial
<220>
   <223> Leptin antisense oligonucleotide
<400> 16
   gcacccaggg ctgaggtcca 20
<210> 17
   <211> 21
   <212> DNA
   <213> Artificial
<220>
   <223> CBFA-1 sense oligonucleotide
<400> 17
   gtggacgagg caagagtttc a 21
<210> 18
   <211> 21
   <212> DNA
   <213> Artificial
<220>
   <223> CBFA-1 antisense oligonucleotide
<400> 18
   tggcaggtag gtgtggtagt g 21
<210> 19
   <211> 28
   <212> DNA
   <213> Artificial
<220>
   <223> PPARgamma2 sense oligonucleotide
<400> 19
   aactgcgggg aaacttggga gattctcc 28
<210> 20
   <211> 26
   <212> DNA
   <213> Artificial
<220>
   <223> PPARgamma2 antisense oligonucleotide
<400> 20
   aataataagg tggagatgca ggctcc 26
<210> 21
   <211> 21
   <212> DNA
   <213> Artificial
<220>
   <223> OCN sense oligonucleotide
<400> 21
   atgagagccc tcacactcct c 21
<210> 22
   <211> 19
   <212> DNA
   <213> Artificial
<220>
   <223> OCN antisense oligonucleotide
<400> 22
   cgtagaagcg ccgataggc 19
<210> 23
   <211> 20
   <212> DNA
   <213> Artificial
<220>
   <223> MyoD sense oligonucleotide
<400> 23
   aagcgccatc tcttgaggta 20
<210> 24
   <211> 20
   <212> DNA
   <213> Artificial
<220>
   <223> MyoD antisense oligonucleotide
<400> 24
   gcgagaaacg tgaacctagc 20
<210> 25
   <211> 20
   <212> DNA
   <213> Artificial
<220>
   <223> SMMHC sense oligonucleotide
<400> 25
   ctgggcaacg tagtaaaacc 20
<210> 26
   <211> 20
   <212> DNA
   <213> Artificial
<220>
   <223> SMMHC antisense oligonucleotide
<400> 26
   tatagctcat tgcagcctcg 20
<210> 27
   <211> 21
   <212> DNA
   <213> Artificial
<220>
   <223> GFAP sense oligonucleotide
<400> 27
   ctgttgccag agatggaggt t 21
<210> 28
   <211> 20
   <212> DNA
   <213> Artificial
<220>
   <223> GFAP antisense oligonucleotide
<400> 28
   tcatcgctca ggaggtcctt 20
<210> 29
   <211> 24
   <212> DNA
   <213> Artificial
<220>
   <223> Nestin sense oligonucleotide
<400> 29
   ggcagcgttg gaacagaggt tgga 24
<210> 30
   <211> 24
   <212> DNA
   <213> Artificial
<220>
   <223> Nestin antisense oligonucleotide
<400> 30
   ctctaaactg gagtggtcag ggct 24
<210> 31
   <211> 21
   <212> DNA
   <213> Artificial
<220>
   <223> SOX9 sense oligonucleotide
<400> 31
   ctctgcctgt ttggactttg t 21
<210> 32
   <211> 21
   <212> DNA
   <213> Artificial
<220>
   <223> SOX9 antisense oligonucleotide
<400> 32
   cctttgcttg ccttttacct c 21
<210> 33
   <211> 20
   <212> DNA
   <213> Artificial
<220>
   <223> Collagen type X sense oligonucleotide
<400> 33
   agccagggtt gccaggacca 20
<210> 34
   <211> 20
   <212> DNA
   <213> Artificial
<220>
   <223> Collagen type X antisense oligonucleotide
<400> 34
   ttttcccact ccaggagggc 20
<210> 35
   <211> 20
   <212> DNA
   <213> Artificial
<220>
   <223> Aggrecan sense oligonucleotide
<400> 35
   cactgttacc gccacttccc 20
<210> 36
   <211> 20
   <212> DNA
   <213> Artificial
<220>
   <223> Aggrecan antisense oligonucleotide
<400> 36
   accagcggaa gtccccttcg 20